# EUROPÄISCHE PATENTSCHRIFT

(11) **EP 1 078 104 B1**
(45) Veröffentlichungstag und Bekanntmachung des Hinweises auf die Patenterteilung: **09.10.2002**
(21) Anmeldenummer: 99938039.7
(22) Anmeldetag: 21.05.1999
(51) Int. Cl.: C12Q 1/68

(54) **NACHWEIS VON ANTIBIOTIKUMRESISTENZEN IN MIKROORGANISMEN**
DEMONSTRATING RESISTANCE TO ANTIBIOTICS IN MICROORGANISMS
MISE EN EVIDENCE DE RESISTANCES A DES ANTIBIOTIQUES DANS DES MICRO-ORGANISMES

(30) Priorität: 22.05.1998 DE 19823098; 13.04.1999 DE 19916610
(43) Veröffentlichungstag der Anmeldung: 28.02.2001
(73) Patentinhaber: Creatogen Aktiengesellschaft, 86156 Augsburg (DE)
(72) Erfinder: HAAS, Rainer, D-81547 München (DE); TREBESIUS, Karlheinz, D-83093 Bad Endorf (DE); APFEL, Heiko, D-86356 Neusäss (DE)
(74) Vertreter: Weiss, Wolfgang, Dipl.-Chem. Dr.
(86) Internationale Anmeldenummer: EP9903527
(87) Internationale Veröffentlichungsnummer: WO99061660

(56) Entgegenhaltungen:
- WO-A-93/22445
- WO-A-95/33074
- WO-A-95/34574
- US-A- 5 571 674
- VERSALOVIC J ET AL.: "Point mutations in the 23S rRA gene of Helicobacter pylori associated with different levels of clarithromycin resistance" JOURNAL OF ANTIMICROBIAL CHEMOTHERAPY, Bd. 40, 1997, Seiten 283-286, XP002119619 in der Anmeldung erwähnt
- AMANN R I ET AL.: "Fluorescent-oligonucleotide probing of whole cells for determinative, phylogenetic, and environmental studies in microbiology" JOURNAL OF BACTERIOLOGY, Bd. 172, Nr. 2, 1990, Seiten 762-770, XP002119620 in der Anmeldung erwähnt
- MOROTOMI M ET AL: "OLIGONUCLEOTIDE PROBE FOR DETECTION AND IDENTIFICATION OF CAMPYLOBACTER PYLORI" JOURNAL OF CLINICAL MICROBIOLOGY, Bd. 27, Nr. 12, 1. Dezember 1989 (1989-12-01), Seiten 2652-2655, XP000605766 ISSN: 0095-1137
- ROSS J I ET AL.: "Clinical resistance to erythromycin and clindamycin in cutanous propionibacteria from acne patients in associated with mutations in 23S rRNA" ANTIMICROBIAL AGENTS AND CHEMOTHERAPY, Bd. 41, Nr. 5, 1997, Seiten 1162-1165, XP002119621 in der Anmeldung erwähnt
- LUCIER T S ET AL.: "Transition mutations in the 23S rRNA of erythromycin-resistant isolates of Mycoplasma pneumoniae" ANTIMICROBIAL AGENTS AND CHEMOTHERAPY, Bd. 39, Nr. 12, 1995, Seiten 2770-2773, XP002119622 in der Anmeldung erwähnt
- MEIER A ET AL.: "Identification of mutations in 23S rRNA gene of clarithromycin-resistant Mycobacterium intracellulare" ANTIMICROBIAL AGENTS AND CHEMOTHERAPY, Bd. 38, Nr. 2, 1994, Seiten 381-384, XP002119623 in der Anmeldung erwähnt
- SIGMUND C D ET AL.: "Antibiotic resistance mutations in ribosomal RNA genes of Escherichia coli" METHODS IN ENZYMOLOGY, Bd. 164, 1988, Seiten 673-690, XP002119624 in der Anmeldung erwähnt
- CANGELOSI G A ET AL.: "Detection of rifampin- and ciprofloxacin-resistant Mycobacterium tuberculosis by using species-specific assays for precursor rRNA" ANTIMICROBIAL AGENTS AND CHEMOTHERAPY, Bd. 40, Nr. 8, 1996, Seiten 1790-1795, XP002119625
- VESTER B UND GARRETT A: "A plasmid-coded and site-directed mutation in Escherichia coli 23S RNA that confers resistance to erythromycin: Implications for the mechanism of action of erythromycin" BIOCHIMIE, Bd. 69, 1987, Seiten 891-900, XP002119626 in der Anmeldung erwähnt
- PINA M ET AL.: "Detection of point mutations associated with resistance of Helicobacter pylori to clarithromycin by hybridization in liquid phase" JOURNAL OF CLINICAL MICROBIOLOGY, Bd. 36, Nr. 11, - 1998 Seiten 3285-3290, XP002119627
- VAN DOORN L-J ET AL.: "Rapid detection, by PCR and reverse hybridization, of mutations in the Helicobacter pylori 23S rRNA gene, associated with macrolide resistance" ANTIMICROBIAL AGENTS AND CHEMOTHERAPY, Bd. 43, Nr. 7, 1999, Seiten 1779-1782, XP002119628

## Beschreibung

Die Erfindung betrifft ein Verfahren zum Nachweis von Makrolid-Antibiotikumresistenzen in Mikroorganismen, insbesondere in Bakterien sowie zur Durchführung des Verfahrens geeignete Reagenzienkits.

Den Grundstein für die Entwicklung einer rRNA-gerichteten in situ-Hybridisierung zum Nachweis von pathogenen Organismen legten Zuckerkandel und Pauling (1965), die in ihrem Artikel : "Molecules as documents of evolutionary history", zum erstenmal auf die Möglichkeit hinwiesen, durch Sequenzvergleiche von Makromolekülen die Evolution der zugehörigen Organismen zu enthüllen. Carl Woese war es dann, der dieses Konzept zur Aufstellung des ersten natürlichen Verwandtschaftssystems bei Prokaryonten benützte (Woese, 1987). Ein weiteres Ergebnis dieser Untersuchungen war, daß rRNA-Sequenzen sogenannte Signatursequenzen aufweisen, die typisch sind für bestimmte Domänen, Phyla, Familien, Gattungen und sogar einzelne Spezies. Der Nachweis dieser Signatursequenzen mit Hilfe von PCR-Primern oder Hybridisierungssonden erlaubt also die Identifikation von Bakterien auf unterschiedlichen taxonomischen Ebenen. Die natürlicherweise vorhandene, hohe Anzahl an rRNA-Molekülen in der bakteriellen Zelle (10⁴-10⁵ in schnell wachsenden Bakterien) erhöht zudem die Sensitivität der Methode und ermöglichte die Anwendung von in situ Hybridisierungstechniken mit rRNA als Zielmolekül. Mit Hilfe radioaktiv markierter Oligonukleotidsonden konnte Giovannoni et al. 1988 als erster rRNA in ganzen bakteriellen Zellen detektieren und DeLong et al. (1989) führte ein Jahr später eine solche in situ Hybridisierung mit fluoreszenzmarkierten Oligonukleotiden durch.

In letzter Zeit ist diese Technik vor allem in der Umweltmikrobiologie häufig eingesetzt worden. Dabei standen die Lokalisation bestimmter physiologischer Gruppen (Wagner et al., 1993; Ramsing et al., 1993) und der Einfluß bestimmter Agentien auf die Populationszusammensetzung eines Ökosystems (Wagner et al., 1995) im Mittelpunkt des Interesses.

Aber auch im Bereich der Lebensmittelhygiene wurde diese Technik erfolgreich zur Detektion von Bakterien eingesetzt (Beimfohr et al., 1993). Ein weiteres Feld der Mikrobiologie, in der die rRNA-gerichtete Ganzzellhybridisierung Anwendung findet, ist die medizinischen Mikrobiologie.

So wurde *H*. *influenzae* in Rachenabstrichen von Kindern detektiert (Forsgren et al., 1994), *Candida* Spezies in Blutkulturen und Gewebeproben von künstlich infizierten Tieren nachgewiesen (Lischewski et al., 1996, Lischewski et al., 1997), pathogene *Yersinia* Spezies in Gewebeschnitten, Stuhl und Rachenproben detektiert (Trebesius et al., 1998) und Salmonella in Abstrichen ebenso erfolgreich hybridisiert (Nordentoftet al., 1997) wie Bifidobakterien in Stuhlproben (Langendijk et al., 1995).

Wie verschiedene Untersuchungen gezeigt haben, hängt die Anzahl der Ribosomen in schnellwachsenden, heterotrophen Bakterien stark von der Wachstumsrate und der physiologischen Aktivität des Organismus ab (Schaechter et al., 1958). Da die Menge an gebundener Sonde proportional zur rRNA-Menge ist, läßt sich indirekt über die hybridisierungsvermittelte Fluoreszenz auch der Wachstumszustand einer Zelle ermitteln (DeLong et al., 1989).

Betrachtet man den Translationsapparat eukaryotischen und bakteriellen Zellen, so ergeben sich erhebliche Unterschiede in Funktion und Aufbau der einzelnen Komponenten. Diese Unterschiede schaffen ein therapeutisches Fenster für eine Reihe von Wirkstoffen, die spezifisch in den bakteriellen, aber nicht in den eukaryontischen Translationsprozess eingreifen. Tabelle 1 zeigt häufig eingesetzte Antibiotika, die in den Translationsprozess der bakteriellen Zelle eingreifen. Nach den Peptidoglycan-gerichteten Antibiotika besitzen diese Wirkstoffe den zweithöchsten weltweiten Marktanteil.

Der massive therapeutische Einsatz dieser Substanzen führt aber auch zum Auftauchen von Resistenzen bei klinischen Isolaten und somit zu Therapieversagen. Für die Entstehung einer solchen Mutation können eine Reihe von Ursachen verantwortlich sein:
(1) Veränderung der Antibiotikazielstelle
(2) Modifikation des Antibiotikas
(3) Veränderung des Antibiotika-Transports

Bei den MLS-Antibiotika (Makrolid, Lincosamid, Streptogramin B), die ihre Wirkung über eine Blockade des ribosomalen Peptidyltransferasezentrums erzielen, lassen Untersuchungen klinischer Isolate den Schluß zu, daß in der überwiegenden Mehrzahl der Fälle Veränderungen der Antibiotikazielstelle für die Resistenzentwicklung verantwortlich sind (Versalovic et al., 1997). Auch dabei sind mehrere Varianten denkbar.
(1) Mutation ribosomaler Proteine
(2) Mutation der rRNA
(3) Posttranskriptionelle Modifikation

Während man früher allgemein annahm, daß die Veränderung ribosomaler Proteine hauptsächlich für die Resistenzentwicklung verantwortlich sei, sprechen experimentelle Daten neueren Ursprungs gegen eine solche Theorie und unterstützen vielmehr die These, daß Veränderungen direkt an der ribosomalen RNA (posttranskriptionelle Methylierung oder Mutation) zur Resistenzentwicklung führen.

**Tabelle 1:**

| Klasse/Wirkstoff | Einsatz |
|---|---|
| **Aminoglycosid-/Aminocyclitol-Antibiotika** (Dihydro-)Streptomycin Neomycin, Paromomycin Kanamycin Gentamicin, Tobramycin, Amikazin, Netilmicin, Sisomicin Spectinomycin | **Marktanteil: 3%** Tuberkulosetherapie; Resistenz häufig orale und topische Anwendung parenterale Gabe; Resistenz häufig neu; breites Spektrum (nicht Streptococcen und Enterococcen; Oto- und Nephrotoxisch; Blut spiegelkontrollieren) penicillinasefeste Gonokokken |
| **Lincosamide** | |
| Lincomycin, Clindamycin | gram positive Bakterien und gram negative Anaerobier; gute Penetration in Knochengewebe; bei Toxinbildnern |
| **Makrolide** | **Marktanteil: 11%** |
| Erythromycin, Roxithromycin, Clarithromycin, Azithromycin | gegen gram positive und gram negative Kokken, Chlamydien sowie Mycolasmen, Helicobacter; gute Membrangängigkeit = = > intrazelluläre Bakterien |
| **Tetracycline** | **Marktanteil: 3,5%** |
| Tetracyclin, Oxytetracyclin, Rolitetracyclin, Doxycyclin, Minocyclin | breites Spektrum, einschließlich Chlamydien und Rickettsien; vorwiegend bakteriostatisch; Resistenz häufig; bei Kleinkindern Ablagerung in den Zähnen; |

So zeigten proteinfreie 23S rRNA-Extrakte in vitro Peptidyltransferaseaktivität, die durch Carbomycin hemmbar war (Noller et al., 1992). Zudem liegen die Affinitätkonstanten für die Bindung zwischen Erythromycin und ribosomalen Proteinen, wie L15, die als potentielle Kandidaten für Resistenzentstehung gehandelt werden, um mehrere Größenordnungen unter der, die für komplette Ribosomen ermittelt wurden (Weisblum, 1995). Vor allem aber weist das Fehlen Erythromycinresistenter, klinischer Isolate, die eine Mutation in ihren ribosomalen Proteinen aufweisen, auf eine eher geringe Bedeutung dieses Resistenzmechanismus hin (Weisblum, 1995).

Häufig anzutreffen sind jedoch die beiden anderen Resistenzmechanismen, wobei auffällt, daß die Zielregion für beide Veränderungen bestimmte Basen der Domäne V der 23S rRNA betreffen (Brimacombe, 1990).

Ein Adeninrest in *E.coli* Position 2058 (Nummerierung nach Brosius et al., 1981), der in der Tertiärstruktur der 23S rRNA zwischen den Helices 73 und 74 lokalisiert ist (Nummerierung nach Brimacombe et al., 1980), ist Substrat für die Modifikation durch Methylasen aus der erm-Familie, die aus verschiedenen Makrolid-resistenten, klinischen Isolaten isoliert wurden (Weisblum, 1995). Auch eine A = > G-Transition in dieser Position führt bei einer Reihe von phylogenetisch unterschiedlichen Bakterien (*Mycobacterium intracellulare* (Meier et al., 1994), *H*. *pylori* (Versalovic et al., 1997), *E*. *coli* (Sigmund et al., 1988, Vester and Garrett, 1987), *Propionibacterium acnes* (Ross et al., 1997), *Mycoplasma pneumoniae* (Lucier et al., 1995) zu einem Resistenz-Phänotyp. Tabelle 2 zeigt eine Reihe weitere Mutationen im Peptidyltransferasezentrum der 23S rRNA, die bei resistenten Bakterien gefunden werden. Die Konserviertheit der Positionen, die zu MLS-Resistenz führen, und deren Auffinden in verschiedensten phylogenetischen Gruppen (nicht nur Bakterien) zeigt die generelle Natur dieses Phänomens an. Da das Auffinden dieser Mutationen relativ neu ist, ist damit zu rechnen, daß in den kommenden Jahren noch bei einer Reihe weiterer klinischer Isolate Modifikationen/Mutationen der rRNA/rDNA als Ursache für Resistenz-entwicklung dingfest gemacht werden. Hierfür gibt es bei den Mollicutes (*Mycoplasma*, *Ureaplasma*) bereits experimentelle Hinweise (Palu et al., 1989, Stopler and Branski, 1986), für die MLS-Antibiotika auch Mittel der Wahl bei der Therapie sind.

**Tabelle 2:**

| **Mutation** | **Spezies** | **Phänotyp** |
|---|---|---|
| G2057A | *P*.*acnes* | Cla^{R},Ery^{R} |
| A2058G | *P.acnes* | Cla^{R},Ery^{R} |
| A2059G | *P*.*acnes* | Cla^{R},Ery^{R} |
| A2058G | *M*.*pneumoniae* | Ery^{R} |
| A2059G | *M.pneumoniae* | Ery^{R} |
| C2611U | *E*.*coli* | Ery^{R}Lin^{R}Sgb^{R} |
| G2032A | *E*.*coli* | Ery^{HS}Cln^{R}Cam^{R} |
| G2032U | *E*.*coli* | Ery^{HS}Cln^{S}Cam^{S} |
| G2032C | *E*.*coli* | Ery^{HS}Cln^{S}Cam^{S} |
| G2057A | *E*.*coli* | Ery^{R},Cam^{R} |
| A2058G | *E.coli* | Ery^{R} |
| A2058U | *E.coli* | Ery^{R} |
| A2058G | *M.intracelluiare* | Cla^{R} |
| A2058C | *M.intracellular* | Cla^{R} |
| A2058U | *M*.*intracellular* | Cla^{R} |
| A2058G | *H.pylori* | Cla^{R} |
| A2058C | *H*.*pylori* | Cla^{R} |
| A2059G | *H*.*pylori* | Cla^{R} |
| A2503C | *E*.*coli* | Cam^{R} |

Tabelle modifiziert nach Referenz Weisblum, 1995. Cam, Chloramphenicol, Cla, Clarithromycin, Cln, Clindamycin, Ery, Erythromycin, Lin, Lincomycin, Sgb, Streptogramin Typ B. HS, Hypersensitiv; R, resistent; S, sensitiv.

In Anbetracht zunehmender Antibiotika-Resistenzen bei klinisch relevanten Bakterien gewinnt die rasche Identifizierung resistenter Bakterien eine immer größere Bedeutung. Die Anzahl pathogener Bakterienarten, die gegen mindestens ein therapeutisch bedeutsames Antibiotikum resistent sind, nimmt kontinuierlich zu. In Anbetracht dieser Situation kann eine wirksame Therapie nur nach Kenntnis des Resistenzstatus des pathogenen Keims durchgeführt werden. Die allgemein praktizierten Verfahren zur Bestimmung des Resistenzstatus von Bakterien beruhen im wesentlichen auf zeitaufwendige Wachstumstests, welche die Wirksamkeit der therapeutisch einsetzbaren Antibiotika untersuchen. Zu diesem Zweck müssen die Bakterien in der Regel zweimal angezüchtet werden, was ca. 48 Stunden dauert. Die Untersuchung langsam wachsender Keime dauert entsprechend länger. Gemäß dem gegenwärtigen Stand der Technik kann somit eine hochwirksame Therapie nur mit einer zeitlichen Verzögerung von mindestens 1 bis 2 Tagen eingeleitet werden.

Die Weiterentwicklung der in situ Hybridisierung zum Nachweis von Punktmutationen liefert einen Lösungsansatz für die rasche Identifizierung von Antibiotika-Resistenzen bei Bakterien, insbesondere für solche Antibiotika, die gegen den Translationsapparat der Mikroorganismen ausgerichtet sind. Die Komponenten des Translationsapparates sind in großer Kopienzahl in einer Zelle vorhanden und können somit direkt, d.h. ohne zusätzlichen Amplifikationsschritt, untersucht werden. Von besonderem Interesse in diesem Zusammenhang ist die rRNA, die nach neueren Erkenntnissen an der Ausprägung von Antibiotika-Resistenzen wesentlich beteiligt ist und somit als Indikator herangezogen werden kann.

In den nachfolgenden Ausführungen wird am Beispiel des klinisch bedeutsamen Bakteriums *Helicobacter pylori* die Vorgehensweise zur Entwicklung von Sonden zur Makrolid-Antibiotikum-Resistenzbestimmung dargestellt und die wesentlichen Bestandteile entsprechender Test-Kits exemplarisch aufgezeigt. Nach diesen Vorgaben kann für jedes klinisch relevante Bakterium und für jedes therapeutisch relevante Antibiotikum ein entsprechender Resistenzbestimmungstest abgeleitet werden.

Die Entwicklung von Antibiotikumresistenzen durch Mutationen ribosomaler Nukleinsäuresequenzen hat auch Konsequenzen für die Behandlung von Helicobacter pylori Infektionen. Das Auftreten von spiralförmigen Bakterien in der menschlichen Magenschleimhaut wurde zwar schon seit Anfang dieses Jahrhunderts beschrieben (Bizzozero, 1893). Die Tatsache, daß es sich dabei um pathogene Keime handelt wurde jedoch erst mit der erfolgreichen Isolierung und Kultivierung dieses Bakteriums durch Marshall und Warren (Warren and Marshall, 1983; Marshall *et al*., 1984) aus der Magenschleimhaut eines Patienten mit einem Magengeschwür (Ulcus ventriculi) realisiert und wissenschaftlich anerkannt. Wie erste Analysen ergaben, handelte es sich bei den isolierten Mikroorganismen um Gram-negative, spiralförmige Bakterien mit extrem hoher Beweglichkeit und der ungewöhnliche Fähigkeit im stark sauren Milieu (bis ca. pH 1) zu überleben. Ursprünglich als *Campylobacter pylori* bezeichnet, wurden die Keime schließlich aufgrund biochemischer und morphologischer Eigenschaften in die neu gegründete Gattung "*Helicobacter*" eingruppiert (Goodwin *et al*., 1989).

1987 haben Dent und Mitarbeiter erstmals einen korkenzieherartigen Organismus in der menschlichen Magenschleimhaut identifiziert und es als "*Gastrospirillum hominis"* bezeichnet. Da die Kultivierung eines solchen Bakteriums bisher erst in Ausnahmefällen gelungen ist, erfolgt die Zuordnung zu dieser "Spezies" aufgrund der Lokalisation in der menschlichen Antrumschleimhaut und der Zellmorphologie (korkenzieherartig gewundenes Stäbchen). Solnick und Mitarbeiter haben 1993 die 16S rRNA-Sequenz von zwei "*Gastrospirillum hominis*"-Stäbchen bestimmt und festgestellt, daß dieses Bakterium dem Genus "*Helicobacter"* zuzuordnen ist. Sie schlugen daraufhin den Namen "*Helicobacter heilmannii*" vor. Bereits in dieser Untersuchung wurde klar, daß es eigentlich nicht gerechtfertigt erschien, diese beiden Stämme unter dem Dach einer Spezies zusammenzufassen, da der Unterschied in der 16S rRNA-Sequenz über 3,5% betrug (ab 3% ist es *arte legis* zwei Isolate als unterschiedliche Spezies zu betrachten), während der Unterschied eines der Stämme (Gh2) zu *Helicobacter felis* nur 1,5% betrug. Ein kultivierbarer *H*.*heilmannii* wurde von Holck et al. 1997 beschrieben. Sequenzierdaten zeigten aber, daß dieser Organismus näher mit dem neu beschriebenen *Helicobacter salomonis* verwandt ist als mit dem von Solnick beschriebenen *H*.*heilmannii*.

"*Helicobacter heilmannii*" (ehemaliges "*Gastrospirillum hominis*") ist daher eine phylogenetisch heterogene Gruppe von *Helicobacter* Spezies, die selten in der humanen Magenschleimhaut gefunden werden, jedoch genau wie *H.pylori* ursächlich, u.a. an der Entwicklung von Magengeschwüren beteiligt ist (Yeomans et al., 1996). Die Zuordnung zu dieser Gruppe erfolgt über morphologische Kriterien (korkenzieherartig gewundene, große Stäbchen).

Schon innerhalb weniger Jahre wurde die Bedeutung der *Helicobacter* Infektion und die Tragweite dieser Entdeckung klar. Epidemiologische Untersuchungen von Taylor und Blaser ( 1991) zeigten, daß die *Helicobacter* Infektion weltweit auftritt, und daß ca. 50 % der Bevölkerung mit diesen Bakterien infiziert ist, wobei die Infektionsrate in den Entwicklungsländern höher ist als in industrialisierten Ländern. Ferner beobachtet man, daß die Wahrscheinlichkeit einer chronischen *Helicobacter* Infektion mit steigendem Lebensalter drastisch zunimmt. Damit zählen *Helicobacter* Infektionen zu den häufigsten chronischen bakteriellen Infektionen des Menschen.

Heute weiß man, daß die Infektion zwangsläufig zur Auslösung einer bakteriellen Gastritis (Typ-B Gastritis) beim Menschen führt. Es gilt ferner als gesichert, daß *Helicobacter* Infektionen auch eine ursächliche Rolle bei der Entstehung von Magen- und Zwölffingerdarmgeschwüren (Ulcus ventriculi und Ulcus duodeni)spielen (Hentschel *et al.,* 1991). Nach einer Studie von Forman et al (Forman *et al*., 1993) führt eine *H. pylori* Infektion zu einem 6 - 12-fach erhöhten Risiko zur Entstehung einiger Formen des Magenkarzinoms (Adenokarzinom), Auch die seltener auftretenden MALT (**M**ucosa **A**ssociated **L**ymphoid **T**issue) Lymphome des Magens, die als Vorstufen von B-Zell-Tumoren des Immunsystems angesehen werden, sind vermutlich eine Folge der *Helicobacter* Infektion. Diesbezüglich konnte gezeigt werden, daß *Helicobacter heilmannii* ein größeres kanzerogenes Potential besitzt als *Helicobacter pylori* und ursächlich für das gastrische MALT-Lymphom verantwortlich ist (Stolte et al., 1997; Regimbeau et al., 1998). Eine antibakterielle Behandlung entsprechender Patienten mit erfolgreicher Eradikation (totaler Elimination) von *Helicobacter* führt sowohl zu einer Abheilung von Magengeschwüren als auch von "low grade" MALT Lymphomen (Sipponen and Hyvärinen, 1993; Isaacson and Spencer, 1993; Stolte and Eidt, 1993).

Bevor die Existenz und die Bedeutung von *Helicobacter spec.* von die Ulkuserkrankungen bekannt waren, wurden diese durch sog. Antazida oder H₂-Rezeptorantagonisten behandelt. Dabei handelt es sich um Substanzen welche die Säuresekretion der Magenparietalzellen inhibieren. Unter dem Einfluß dieser Arzneimittel kommt es meist zur Abheilung von Geschwüren, da jedoch eine der Ursachen dieser Geschwüre, nämlich die *Helicobacter* Infektion, damit nicht eliminiert wird, kommt es in den meisten Fällen nach kurzer Zeit zu einem erneuten Auftreten der Ulzeration (Rezidiv).

Eine weitere häufig angewandte Therapie bei Ulzerationen ist die Wismut-Behandlung. Verschiedene Wismutsalze (CBS, BSS) haben einen bakteriziden Effekt auf *Helicobacter.* Eine totale Eradikation des Keims wird jedoch nur in 8 - 32 % der Fälle erreicht. Die Behandlung führt anscheinend zu einer vorübergehenden Suppression des Keims, aber nach Absetzen der Behandlung kommt es in den meisten Fällen wieder zum Aufflackern der Infektion, Eine längerdauernde Therapie mit hohen Dosen führt zu einer Akkumulation der Substanz in Leber, Niere und im Nervensystem und hat beträchtliche neurologische Nebenwirkungen (Malfertheiner, 1994).

Seit der Erkenntnis, daß es sich bei den gastroduodenalen Ulkuserkrankungen um Infektionskrankheiten handelt, ist ein Ziel der Behandlung die Eradikation der Erreger durch Antibiotika. Die Monotherapie mit verschiedenen Antibiotika (Amoxycillin, Tetrazyklin, Nitrofuran, Furazolidin, Erythromycin u.a.) stellte sich jedoch als nicht zufriedenstellend heraus, da es auch hier nur bei 0 - 15 % der Behandlungen zur Eradikation der Keime kommt. Auch die früher empfohlenen Dual-Therapien mit einem Säureblocker und einem Antibiotikum, wies eine hohe Versagerquote auf (Malfertheiner, 1994).

Die erfolgreichste Behandlung wird zur Zeit durch eine Kombination eines Säureblockers (z.B. Omeprazol) mit zwei Antibiotika in Form der "französischen" Tripeltherapie (Amoxicillin und Clarithromycin) oder bei Penicillin-Allergie der "italienischen" Tripeltherapie (Clarithromycin und Metronidazol) erreicht, die zu Eradikationsraten von 80-95 % führen kann. Clarithromycin wird aufgrund seiner günstigen Eigenschaften immer mehr zum Mittel der Wahl (Graham, 1995). Bei Therapieversagen wegen Clarithromycin- bzw. Metronidazol-Resistenz der Bakterien wird eine Vierfachtherapie empfohlen (Protonenpumpenhemmer, Wismutsalz, Tetrazyklin und Metronidazol/oder Clarithromycin). Dises Schema verspricht Erfolgsraten um 95%, ist allerdings auch mit erheblichen Nebenwirkungen behaftet.

Eine wichtige Voraussetzung für den Erfolg der Therapie stellt der Antibiotika-Resistenz-Status der Bakterien dar. Sobald eine Resistenz gegen nur eines der beiden eingesetzten Antibiotika vorliegt, tritt eine stark erhöhte Versagerquote bei der Behandlung auf (Buckley et al., 1997). Anlaß zur Beunruhigung gibt allerdings die Beobachtung, daß die Anzahl der Metronidazol- und Clarithromycin-resistenten *H*. *pylori* Isolate in jüngerer Zeit stetig ansteigt. Der Grund dafür ist nicht bekannt, könnte aber in der zunehmenden Anzahl der *H*. *pylori* Eradikationstherapien liegen. Speziell die in der jüngeren Vergangenheit häufig durchgeführten Dualtherapien mit einem Antibiotikum bei denen häufig Resistenzen auftreten. Eine Resistenz gegen Makrolid-Antibiotika wie Clarithromycin oder Erythromycin beruht auf einer Punktmutation in einer bestimmten Regionen der 23S-rRNA. Solche Mutationen treten anscheinend spontan auf und können durch entsprechende Selektion mit einem Antibiotikum leicht isoliert werden. Eine schnelle und zuverlässige Bestimmung des Resistenz-Status der *Helicobacter* Isolate vor der Therapie ist deshalb in Zukunft von großer Bedeutung.

Die einfachste Methode, eine akute *Helicobacter* Infektion relativ sicher nachzuweisen, ist der sogenannte Atemtest (Desroches et al., 1997). Hierbei wird die Zersetzung von oral verabreichtem, ¹³C/¹⁴C-markiertem Harnstoff in ¹³CO₂ oder ¹⁴CO₂ und NH₄ durch die bakterielle Urease gemessen. Das ¹³CO₂ oder ¹⁴CO₂ gelangt über die Blutzirkulation in die Lungen, wird dort über den natürlichen Gasaustausch freigesetzt und somit in der Atemluft meßbar. Die meßtechnische Auswertung der Atemluft erfolgt in einem besonderen Gerät, mit dem nur Speziallabors ausgestattet sind, so daß die Befunderhebung oftmals örtlich getrennt von der Probenentnahme erfolgt und somit zeitlich verzögert ist. Andere nichtinvasive Verfahren basieren auf PCR-Reaktionen, die mit Stuhl oder Speichelproben durchgeführt werden (Schwarz et al., 1997). Diese Verfahren besitzen zwar eine sehr hohe Empfindlichkeit, allerdings ist das Auftreten falsch-positiver Befunde im Routinebetrieb erfahrungsgemäß sehr häufig. Weiterhin wird eine apparative und molekularbiologische Grundausstattung benötigt, welche in einer allgemeinen Arztpraxis nicht vorhanden ist. D.h. auch in diesem Fall müssen die Proben in Speziallabors eingeschickt werden, wodurch eine rasche Befunderhebung ausgeschlossen ist. Mittelbare Verfahren, die im Serum oder Speichel gegen Helicobacter gerichtete Antikörper nachweisen, haben wiederum den Nachteil, daß eine akute Infektion nicht zweifelsfrei nachgewiesen werden kann.

Liegt ein positiver Atemtestbefund vor, wird in der Regel eine Gastroskopie durchgeführt. Oftmals geschieht dies auch bei negativem Befund, insbesondere wenn nach konventioneller Therapie mit Protonenblocker die Symptome nicht abklingen. In der Regel werden bei einer Gastroskopie Gewebeproben entnommen, insbesondere wenn auffällige Veränderungen, wie Entzündungen oder Geschwüre, erkennbar sind. Diese Gewebeproben werden histologisch auf gut- oder bösartige Gewebeveränderungen untersucht. Vor allem werden mikrobiologische Untersuchungen durchgeführt, die den Keim eindeutig zuordnen sollen und seinen Minimale Hemmkonzentration (MHK) Wert bestimmen. Diese Ergebnisse erlauben eine gezielte Therapieplanung und somit eine hohe Erfolgsgarantie. Zur Durchführung der mikrobiologischen Untersuchungen ist eine Anzüchtung des Keims erforderlich, die mehrere Tage in Anspruch nehmen kann. Danach wird eine ebenfalls zeitaufwendige MHK-Wertbestimmung durchgeführt, die eine Kultivierung des Keims erforderlich macht. Durch diese Untersuchung wird der Therapiebeginn wesentlich verzögert, so daß viele Ärzte auf diese Untersuchung verzichten, was zu Lasten des Patienten geht. Dies hat zur Folge, daß bei einer Therapie zu hohe Antibiotika-Dosierungen angesetzt werden, die mit massiven Nebenwirkungen einhergehen. Im anderen Fall kann die Antibiotika-Dosierung zu niedrig angesetzt sein, da der Nachweis Antibiotikaresistenter Keime nicht durchgeführt wurde. Eine schnelle und zuverlässige Identifizierung des möglicherweise krankheitsverursachenden Keims und die unmittelbare Bestimmung seines Resistenzstatus ist deshalb von größter Bedeutung.

In der Literatur sind kokkoide Formen von *H*.*pylori* mehrfach beschrieben. Die klinische Bedeutung dieser Stadien wird allerdings kontrovers diskutiert. Einige Autoren postulieren, daß kokkoide Formen eines morphologische Manifestation des Zelltodes darstellen und nicht mehr zu vegetativen Form revertieren können (Kusters et al., 1997). Andere Gruppen gehen davon aus, daß diese Bakterien lebend, aber nicht kultivierbar sind (VNC, viable but non-culturable). Infektionsexperimente mit verschiedenen Tiermodellen konnten die Frage einer möglichen Aktivierung der kokkoiden Form in die vegetative Form nicht eindeutig klären. Eaton und Mitarbeiter erhielten eine erfolgreiche Infektion von Mini-Schweinen mit vegetativen *H.pylori*, während kokkoide Formen in diesem Modell keine Infektion zeigten (Eaton et al., 1995). Im Gegensatz dazu wurde von zwei unabhängigen Arbeitsgruppen über eine erfolgreiche Infektion mit VNC *H*.*pylori* im Mausmodell berichtet (Cellini et al., 1994; Wang et al., 1997).

Der direkte Nachweis von kokkoiden Formen im menschlichen Magen wurde von Chan et al. anhand von Magengewebeschnitten aus Biopsiematerial erbracht. In 82,8% (53/64) der untersuchten Biopsieproben konnten die Autoren kokkoide Formen von *H*.*pylori* mit einer Hematoxylin-Eosin Färbung nachweisen (Chan et al., 1994). Von Cao et al. wurde ein monoklonaler Antikörper zum spezifischen Nachweis von kokkoiden *H.pylori* im Gewebeschnitt benutzt. Auch hier wurden neben den vegetativen Formen in 100% der Antrumbiopsien (9/9) *H*.*pylori* kokkoide Formen nachgewiesen (Cao et al., 1997).

Weitere Arbeiten zeigen, daß kokkoide Formen bevorzugt durch O₂-Streß und durch Gabe von Antibiotika (Wismut Subcitrat, Erythromycin, Amoxicillin, Metronidazol) induziert werden können (Donelli et al., 1998; Bode et al., 1993; Sorberg et al., 1996; Berry et at., 1995), wobei offensichtlich Polyphosphate als Energiedepot genutzt werden, das für mindestens 3 Monate ausreichen könnte (Bode et al., 1993). Die Bindung an Epithelzellen und die Fähigkeit zur Signaltransduktion, (IL-8-Induktion, Rearrangement des Zytoskeletts, Bindung von Plasminogen, Laktoferrin und Vitronectin auf der Bakterienoberfläche) scheint bei der kokkoiden Form vergleichbar zu den vegetativen Formen erhalten zu sein (Khin et al., 1996; Segal est al., 1996). Es scheint wahrscheinlich, daß die kokkoide Form eine Überdauerungsform des Helicobacter darstellt.

Die Erfindung betrifft eine neuartige Methode zur Bestimmung von Makrolid- Antibiotikumresistenzen bei Mikroorganismen, insbesondere bei Bakterien, die auf veränderte Nukleinsäuresequenzen, insbesondere in ribosomalen Nukleinsäuren beruhen. Bei Verwendung von Hybridisierungssonden, die spezifisch für eine mit Makrolid-Antibiotikumresistenzen assoziierte Nukleinsäuresequenz sind, können überraschenderweise in einem in situ Nachweis-verfahren aufgrund des Auftretens oder des Ausbleibens einer Hybridisierung schnelle und zuverlässige Aussagen über das Vorliegen oder/und die Stärke einer Antibiotikumresistenz bzw. die minimale Hemmkonzentration eines Antibiotikums bei Mikroorganismen aus einer biologischen Probe getroffen werden. Die vorliegende Erfindung ermöglicht eine unvergleichlich rasche und gezielte Befunderhebung für den behandelnden Arzt, was eine hohe Therapiesicherheit für den Patienten bewirkt und eine erhebliche Kostenreduzierung der Gesamtbehandlung ermöglicht.

Ein Gegenstand der vorliegenden Erfindung ist somit ein Verfahren zum Nachweis von Makrolid-Antibiotikumresistenzen bei Mikroorganismen, umfassend die Schritte:
a) Bereitstellen einer Mikroorganismen enthaltenden Probe,
b) Inkontaktbringen der Probe mit mindestens einer Hybridisierungssonde, die spezifisch.für eine mit Makrolid-Antibiotikumresistenzen assoziierte Nukleinsäuresequenz in Mikroorganismen ist, unter Bedingungen, die eine spezifische Hybridisierung der Sonde erlauben und
c) Auswerten der Probe in situ durch Bestimmung des Auftretens oder des Ausbleibens einer Hybridisierung.

In einer besonders bevorzugten Ausführungsform werden beim erfindungsgemäßen Verfahren mehrere unterschiedliche Hybridisierungssonden in Form eines Gemisches eingesetzt. Überraschenderweise binden diese unterschiedlichen Hybridisierungssonden unter identischen Hybridisierungsbedingungen mit ausreichender Spezifität an unterschiedliche Zielsequenzen, um eine Sequenzabweichung von nur einer einzigen Base nachweisen zu können.

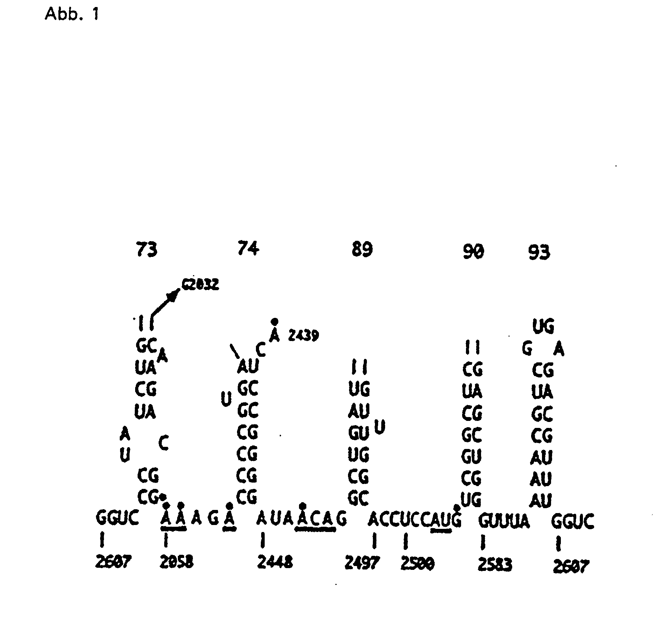

Vorzugsweise wird das erfindungsgemäße Verfahren zum Nachweis von Makrolid-Antibiotikumresistenzen bei bakteriellen Keimen verwendet. Die mit Makrolid-Antibiotikumresistenzen assoziierte Nukleinsäuresequenz wird insbesondere aus ribosomalen Nukleinsäuresequenzen, besonders bevorzugt aus bakteriellen 23S ribosomalen Nukleinsäuresequenzen ausgewählt. Besonders bevorzugt werden Nukleinsäuresequenzen aus dem Peptidyltransferase-zentrum auf der 23S RNA verwendet, dessen Sequenz in Abbildung 1 (für E.coli) dargestellt ist. Besonders bevorzugt umfaßt die ausgewählte Nukleinsäuresequenz einen Bereich entsprechend einem oder mehreren der Nukleotide 2032, 2057, 2058, 2059, 2503 und 2611 auf der E.coli 23S rRNA (Nummerierung nach Brosius et al. 1981), wobei sich Mutationen an diesen Positionen auf den Nachweis von Resistenzen gegen Makrolid-Antibiotika, z.B. Clarithromycin und Erythromycin beziehen.

Darüber hinaus ist das erfindungsgemäße Verfahren jedoch auch zum Nachweis von Makrolid-Antibiotikumresistenzen bei anderen Mikroorganismen, z.B. bei Protozoen, geeignet. Insbesondere kommen Organismen in Betracht, die durch Gabe von Makrolid-Antibiotika bekämpft werden können, z.B. Giardia lamblia, ein im oberen Dünndarm des Menschen vorkommender protozoischer Erreger der Lamblienruhr (Jablonowski et al., 1997), oder Pneumocystis carinii, ein protozoischer Organismus, der bei immundefizienten Patienten, z.B. bei HIV-Patienten, eine oft letal verlaufende Pneumonie auslösen kann.

Gegenüber bekannten Verfahren zum Nachweis von Makrolid-Antibiotikumresistenzen weist das erfindungsgemäße Verfahren eine Reihe von Vorteilen auf. So können - im Gegensatz zu klassischen biochemischen Detektionsverfahren -durch das erfindungsgemäße Verfahren auch langsam wachsende oder in vitro schwierig oder nicht kultivierbare Pathogene schnell untersucht werden. Darüber hinaus ist aufgrund der in situ Detektion eine direkte Lokalisierung der Keime in betroffenen Gewebearealen möglich. Gegenüber anderen molekularbiologischen Verfahren wie PCR zeichnet sich das erfindungsgemäße Verfahren dadurch aus, daß zeit- und arbeitsintensive DNA-Präparationsverfahren wegfallen und daß die Nachweismethode gegenüber evtl. vorhandenen Inhibitoren im Probenmaterial weniger empfindlich ist. Darüber hinaus kann die nachgewiesene Nukleinsäure mit bakteriellen Morphologien, wie z.B. Kokken, Stäbchen etc., assoziiert werden, was zu einer Verbesserung der Verläßlichkeit des Verfahrens führt. Außerdem ist eine Lokalisierung und Quantifizierung des Pathogens in betroffenen Arrealen möglich.

Gegenüber anderen mikroskopischen Verfahren, z.B. Färbeverfahren (Gram, Grocott-Gomori, Giemsa), zeichnet sich das erfindungsgemäße Verfahren durch eine höhere Spezifität und Sensitivität aus. Gegenüber Immunfluoreszenzverfahren ist die geringere Größe der Sonde von Vorteil, die eine bessere Penetration ins Gewebe erlaubt, kaum unspezifische Bindung zeigt sowie universell anwendbar und mit geringeren Kosten verbunden ist.

Das erfindungsgemäße Verfahren ist für alle Mikroorganismen geeignet, insbesondere pathogene Bakterien, wie Streptokokken, *Bordetella, Corynebacterium,* vor allem für problematische Mikroorganismen, wie *Helicobacter spec.,* z.B. *Helicobacter heilmannii* oder *Helicobacter pylori*, Mycobakterien, *Porphyromonas gingivalis*, *Propionibacterium acnes, Borrelia burgdorferi*, Mycoplasmen, Chlamydien und *Tropheryma whippelii* sowie Vertreter der Gattungen *Bartonella, Legionella, Norkardia* und *Actinomyces,* aber auch für andere pathogene Keime, wie *Pneumocystis carinii* und *Giardia lamblia*.

Gemäß Schritt (a) des erfindungsgemäßen Verfahrens wird eine Mikroorganismen enthaltende Probe, vorzugsweise im Rahmen einer human- oder tiermedizinischen Befunderhebung, bereitgestellt. Diese Probe kann eine beliebige biologische Probe sein, vorzugsweise stammt sie jedoch aus menschlichen oder tierischen Geweben oder Körperflüssigkeiten, z.B. Gewebeschnitten, Biopsien oder Blutproben. Das erfindungsgemäße Verfahren hat überraschenderweise eine derart hohe Spezifität und Sensitivität, daß die Probe ohne vorherige Kultivierung bzw. Vermehrung der Mikroorgansimen untersucht werden kann.

Besonders bevorzugt wird die Probe vor der Untersuchung mit einem Presumptive-Medium versetzt. Dieses Presumptive-Medium ist ein Medium, dessen Zusammensetzung auf die mikrobielle Flora der klinischen Probe abgestimmt ist, insbesondere auf die eventuell vorhandenen krankheitsverursachenden Keime, und diese über einen begrenzten Zeitraum, z.B. von mehreren Stunden bis mehreren Tagen, lebensfähig konserviert, aber das Wachstum der Keime weitgehend unterdrückt. Ein solches Presumptive-Medium besteht im wesentlichen aus einer speziellen Nährlösung, die gegebenenfalls in einer halbfesten organischen Matrix, z.B. einer Agar-Matrix, vorliegt (Westblom et al., 1991). Die Nährlösung beinhaltet eine Stickstoffquelle und essentielle Komponenten, welche die Stabilität der isolierten Keime verbessern, wie etwa Spurenelemente, z.B. Eisen, Zink, Mangan, Vitamine etc. Alle weiteren Bestandteile dieses Presumptive-Mediums liegen vorzugsweise in einer gepufferten wäßrigen Lösung vor. Bestandteile der Stickstoffquelle sind chemische oder proteolytische Aufschlüsse von Proteinen mikrobieller, tierischer oder pflanzlicher Herkunft, wie z.B.: Peptone, Tryptone oder Casitone oder Gemische von diesen, in Abhängigkeit der Anforderungen der ausgewählten mikrobiellen Flora. Für anaerobe bzw. mikroaerophile Keime werden dem Medium reduzierende Substanzen, wie z.B. reduziertes Cystein oder Thioglykolat, zugemischt oder/und Sauerstoff-abweisende Zusätze beigefügt.

Gegebenenfalls kann die Probenvorbereitung zusätzlich mit einem Anreicherungsverfahren kombiniert werden, insbesondere bei der Isolierung von Keimen aus flüssigen Proben, z.B. Blut- oder Urinproben. Das bevorzugte Anreicherungsverfahren beruht auf einer Bindung der Keime an einer festen Matrix, insbesondere auf der Grundlage elektrostatischer Wechselwirkungen, z.B. an Aktivkohle, oder über definierte Liganden, wie z.B. Polylysin, die an eine feste Matrix gebunden sind. Gemäß dem Verfahren fließt das Blut bei der Entnahme in ein abgeschlossenes Gefäß, z.B. eine Spritze, das diese Matrix enthält. Nach einer kurzen Inkubationszeit wird das Blut durch das o.g. Presumptive-Medium ausgetauscht, wobei die Matrix-adsorbierten Keime im abgeschlossenen Gefäß lebensfähig konserviert werden.

Das Presumptive-Medium kann gegebenenfalls mit Nachweisreagenzien, z.B. Indikatoren, versetzt werden, die das Vorhandensein von pathogenen Leitkeimen innerhalb kurzer Zeit, d.h. innerhalb von Minuten anzeigen. Dieser Indikator kann z.B. ein Substratgemisch für ein sezerniertes Leitenzym (z.B. für die *Helicobacter* Urease) sein, dessen Umsetzung zu einem chromogenen Produkt führt oder/und die Produktion charakteristischer Substanzen wie etwa bestimmte Toxine oder Metaboliten anzeigt.

Vor der Untersuchung auf Antibiotikumresistenzen wird die Probe weiterhin vorzugsweise fixiert, z.B. mit Formaldehyd, Paraformaldehyd oder Glutardialdehyd und vorzugsweise permeabilisiert, um ein besseres Eindringen der Hybridisierungssonden in die Zellen zu erlauben. Das Permeabilisierungsverfahren wird auf das jeweils untersuchte Pathogen ausgerichtet, wobei für Gram-negative Bakterien und Gram-positive Bakterien jeweils geeignete Permeabilisierungsverfahren verwendet werden müssen.

Die Hybridisierungssonden können Nukleinsäuren wie DNA, RNA, Nukleinsäureanaloga oder Kombinationen davon sein. Vorzugsweise werden die Hybridisierungssonden aus Nukleinsäuren wie DNA oder Nukleinsäureanaloga wie peptidischen Nukleinsäuren (PNA) ausgewählt.

Die Hybridisierungssonden weisen einen Hybridisierungsbereich auf, der mit einer Nukleinsäurezielsequenz des Mikroorganismus selektiv hybridisieren kann. Vorzugsweise hat dieser Hybridisierungsbereich eine Länge entsprechend 10 bis 30 Nukleotidbausteinen, besonders bevorzugt 15 bis 20 Nukleotidbausteinen, insbesondere 17 bis 18 Nukleotidbausteinen. Die beim erfindungsgemäßen Verfahren verwendeten Hybridisierungssonden eignen sich insbesondere zum Nachweis von Mutationen, z.B. Mutationen von einzelnen Nukleotiden oder kurzen Nukleotidabschnitten, die ausgewählt sind aus Deletionen, Transversionen, Transitionen und Modifikationen, z.B. Methylierungen, der entsprechenden Wildtypsequenz.

Das erfindungsgemäße Verfahren erlaubt die Verwendung einer einzigen Hybridisierungssonde. In vielen Fällen hat es sich jedoch als günstig erwiesen, eine Kombination von mehreren Hybridisierungssonden zu verwenden, die spezifisch für unterschiedliche mit Makrolid-Antibiotikumresistenzen assoziierte Nukleinsäuresequenzen sind. Beispiele für erfindungsgemäße Hybridisierungssonden, mit denen der Nachweis von Makrolidresistenzen an den Positionen 2058 und 2059 der 23S RNA möglich ist, sind die Sonden ClaR1 (SEQ ID NO. 1), ClaR2 (SEQ ID NO. 2) und ClaR3 (SEQ ID NO. 3). Diese Sonden können sowohl einzeln als auch in Kombination eingesetzt werden.

Um die Sensitivität des erfindungsgemäßen Verfahrens zu erhöhen, können die für mit Makrolid-Antibiotikumresistenzen assoziierten Mutationen spezifischen Hybridisierungssonden in Kombination mit einer oder mehreren Hybridisierungssonden eingesetzt werden, die spezifisch für eine mit dem Wildtyp des Mikroorganismus (d.h. einem Antibiotikum-sensitiven Stamm) assoziierte Nukleinsäuresequenz sind. Beispiel für eine derartige Hybridisierungssonde ist ClaWT (SEQ ID NO. 4), die beispielsweise in Kombination mit den zuvor genannten Sonden ClaR1, ClaR2 und ClaR3 verwendet werden kann.

Bei Verwendung von Wildtyp-spezifischen Sonden und Makrolid-Antibiotikumresistenzen Mutanten-spezifischen Sonden, die jeweils unterschiedliche Markierungsgruppen tragen, kann das Verhältnis von resistenten zu sensitiven Keimen in einer Probe bestimmt werden.

Darüber hinaus kann beim erfindungsgemäßen Verfahren noch zusätzlich eine Hybridisierungssonde verwendet werden, die spezifisch für eine Spezies oder Gattung von Mikroorganismen ist. Derartige Hybridisierungssonden sind bekannt und vorzugsweise spezifisch gegen ribosomale Nukleinsäuresequenzen, z.B. 23S RNA, 16S RNA oder ribosomale Spacersequenzen, gerichtet. Beispiele für solche Sonden sind Hybridisierungssonden, die gegen Sequenzen aus *H*.*pylori* 16S rRNA gerichtet sind, die homolog zu den E.coli Regionen 110-140 oder/und 740-780 sind, insbesondere die zum Spezies-spezifischen Nachweis von *Helicobacter pylori* verwendeten Hybridisierungssonden Hpy1-16S-753 (SEQ ID NO. 5) und 120b (SEQ ID NO. 6), 585 (SEQ ID NO. 7) und 219 (SEQ ID NO. 8). Weitere Beispiele für solche Sonden, die spezifisch gegen Sequenzen aus *H*.*heilmanii* 16S rRNA gerichtet sind, die homolog zu den *E.coli* Regionen 580-610 oder/und 640-670 sind, insbesondere die zum Spezies-spezifischen Nachweis von Vertretern der *H.heilmannii*-Gruppe verwendeten Hybridisierungssonden Hh1 (SEQ ID NO. 9), Hh2 (SEQ ID NO. 10), Hh3 (SEQ ID NO. 11) und Hh4 (SEQ ID NO. 12).

Vorzugsweise werden beim erfindungsgemäßen Verfahren Hybridisierungssonden verwendet, die eine Direktmarkierung tragen, d.h. eine oder mehrere Markierungsgruppen sind direkt, vorzugsweise durch kovalente Bindung mit der Sonde verknüpft. Andererseits können auch indirekt markierte bzw. markierbare Sonden verwendet werden, z.B. Sonden, die eine Biotingruppe tragen, die wiederum über die Bindung von Streptavidin nachgewiesen wird, wobei das Streptavidin mit einer geeigneten Markierungsgruppe verknüpft ist. Alternativ können die Hybridisierungssonden auch nicht mit Nukleinsäuresequenzen des Mikroorganismus hybridisierende Sequenzbereiche enthalten, die zur Hybridisierung mit einer weiteren komplementären (direkt oder indirekt) markierten Sonde eingesetzt werden können.

Die für das erfindungsgemäße Verfahren verwendeten Markierungsgruppen sind an sich beliebig, sofern sie einen ausreichend sensitiven in situ Nachweis ermöglichen. Bevorzugt sind Farbstoff-, Fluoreszenz- oder/und Enzymgruppen. Besonders bevorzugt sind Fluoreszenzmarkierungsgruppen.

Werden beim erfindungsgemäßen Verfahren mehrere Arten von Hybridisierungssonden (z.B. mehrere Mutations-spezifische Sonden, eine oder mehrere Mutations-spezifische Sonden in Kombination mit Wildtyp-spezifischen Sonden oder/und Spezies-spezifischen Sonden) verwendet, kann es günstig sein, unterschiedliche, d.h. nebeneinander nachweisbare Markierungsgruppen einzusetzen. So kann man beispielsweise beim zusätzlichen Einsatz Spezies-spezifischer Sonden, die eine von der Markierung der Mutations-spezifischen Sonde unterschiedliche Markierung aufweisen, bei gleichzeitiger Hybridisierung beider Sonden eine dritte unterschiedliche Farbgebung erhalten, die sich aus der Mischung beider Sondenfarben ergibt. Wird z.B. für die Sonden zur Bestimmung der Bakterienspezies ein grünes Fluorophor, z.B. Fluorescein, gewählt, und für die Sonden zur Bestimmung für die Makrolid-Antibiotikumresistenz ein rotes Fluorophor, z.B. Rhodamin, ergibt sich aus beiden Mischfarben gelb.

Aufgrund dieser Sondenkombination läßt sich innerhalb einer klinischen Probe gleichzeitig die Identität des Mikroorganismus und das evtl. Vorhandensein einer Makrolid-Antibiotikumresistenz bestimmen. Wenn der nachgewiesene Keim Antibiotika-sensitiv ist, erlaubt die alleinige Bindung der Spezies-spezifischen Sonde eine zuverlässige Identifizierung.

Darüber hinaus ermöglicht das erfindungsgemäße Verfahren sogar eine zusätzliche Aussage über die minimale Hemmkonzentration (MHK) von Makrolid-Antibiotikum resistenten Mikroorganismen. So wurde gefunden, daß bestimmte Punktmutationen bzw. bestimmte Kombinationen von Punktmutationen direkt mit den durch konventionelle Mittel bestimmten MHK-Werten korrelieren. Durch den Einsatz von Hybridisierungssonden, die die Detektion bestimmter Punktmutationen bzw. bestimmter Kombinationen davon erlauben, kann auf diese Weise eine Bestimmung von MHK-Werten vorgenommen werden. Beispielsweise können Hybridisierungssonden für niedrige, mittlere und hohe MHK-Werte speziell hergestellt und eingesetzt werden. Auf diese Weise kann der behandelnde Arzt eine genaue Dosierung des Antibiotikums für die Therapie vornehmen.

Die erfindungsgemäße Hybridisierungssonde ist spezifisch für eine mit Makrolid-Antibiotikumresistenzen assoziierte Nukleinsäuresequenzin Mikroorganismen. Dies bedeutet, es existieren Hybridisierungsbedingungen, bei denen die Hybridisierungssonde an eine mit Makrolid-Antibiotikumresistenzen assoziierte Nukleinsäuresequenz hybridisiert, nicht aber an eine entsprechende Nukleinsäuresequenz aus einem Antibiotikum-sensitiven Wildtyp-Organismus. Für einen bestimmten Test geeignete Hybridisierungsbedingungen, die eine ausreichende Spezifität der Unterscheidung zwischen Wildtyp- und mutierten Sequenzen erlauben, können vom Fachmann ohne weiteres durch empirische Untersuchungen abhängig von der Basenfolge der Hybridisierungssonde und der Zielsequenz ermittelt werden. Als Hybridisierungspuffer wird vorzugsweise ein Puffer verwendet, der 0,5 bis 1,5 M Salz, z.B. NaCI, ein Detergenz, SDS und Formamid enthält. Gewaschen wird vorzugsweise in einem Formamid-freien Puffer. Die Sonden werden derart gewählt, daß die Hybridisierungstemperatur vorzugsweise im Bereich von 45 bis 55°C liegt.

Die Auswertung der Probe erfolgt in situ, vorzugsweise durch mikroskopische Methoden, z.B. mit einem Fluoreszenzmikroskop.

Ein weiterer Gegenstand der Erfindung ist die Verwendung eines in situ Nukleinsäure-Hybridisierungsverfahrens zum Nachweis von Makrolid-Antibiotikumresistenzen in Mikroorganismen, insbesondere in Bakterien. Vorzugsweise wird das Nukleinsäure-Hybridisierungsverfahren wie zuvor beschrieben durchgeführt.

Besonders bevorzugt werden Resistenzen gegen Makrolid-Antibiotika ausgewählt aus der Gruppe bestehend aus Clarithromycin, Erythromycin, Azithromycin und Roxithromycin nachgewiesen.

Noch ein weiterer Gegenstand der vorliegenden Erfindung ist ein Reagenzienkit zum Nachweis von Makrolid-Antibiotikumresistenzen in Mikroorganismen. In einer ersten Ausführungsform beruht dieser Reagenzienkit auf einem Nachweis durch in situ Hybridisierung und umfaßt:
(a) Mittel zur Probenvorbereitung und
(b) mindestens eine Hybridisierungssonde, die spezifisch für eine mit Makrolid-Antibiotikumresistenzen assoziierte Nukleinsäuresequenz in Mikroorganismen ist, und gegebenenfalls mindestens ein Mittel zur Typisierung von Mikroorganismen, z.B. mindestens eine Hybridisierungssonde, die spezifisch für eine Spezies oder Gattung von Mikroorganismen ist.

Vorzugsweise umfassen die Mittel zur Probenvorbereitung ein Presumptive-Medium, das eine Zusammensetzung wie zuvor angegeben aufweisen kann. Alternativ oder zusätzlich können die Mittel zur Probenvorbereitung auch Anreicherungsmittel für Mikroorganismen, z.B. eine Adsorptionsmatrix zur Anreicherung von Keimen aus einer flüssigen Probe, Lösungen oder Suspensionen zur Fixierung der Probe, Hybridisierungs- oder/und Waschpuffer umfassen. Die Hybridisierungssonden sind vorzugsweise wie zuvor beschrieben markiert.

Gemäß einer zweiten Ausführungsform umfasst der Reagenzienkit zum Nachweis von Makrolid-Antibiotikumresistenzen in Mikroorganismen
(a) ein Presumptive-Medium für Mikroorganismen und
(b) mindestens eine Hybridisierungssonde, die spezifisch für eine mit Makrolid-Antibiotikumresistenzen assoziierte Nukleinsäuresequenz in Mikroorganismen ist und gegebenenfalls Mittel zur Typisierung von Mikroorganismen.

Gemäß dieser Ausführungsform sind die Mittel zur Typisierung nicht auf die Verwendung von Hybridisierungssonden und anderen Hybridisierungsreagenzien beschränkt. Denkbar sind auch Indikatorsubstanzen, die das Vorhandensein von Mikroorganismen anzeigen, z.B. Ureasenachweis-reagenzien für *Helicobacter spec.,* z.B. *H*.*heilmannii* oder/und *H.pylori*, Haemolysinreagenzien für Streptokokken, Nachweisreagenzien für Toxine von *Clostridium difficile*, *Corynebacterium diphtheriae*, *Bordetella pertussis*. Vorzugsweise sind die Typisierungsreagenzien für Mikroorganismenspezies oder-gattungen bereits im Presumptive-Medium gelöst oder suspendiert, so daß ein Presumptive-Medium mit kombiniertem Indikatorsystem vorliegt. Außerdem ist denkbar, daß das Presumptive-Medium mit kombiniertem Indikatorsystem zusätzlich mit einem Anreicherungsverfahren kombiniert wird. In diesem Fall werden die Keime in einem ersten Schritt aus Körperflüssigkeiten, wie z.B. Blut, angereichert und in einem zweiten Schritt mit dem Indikator-haltigen Presumptive-Medium versetzt. Darüber hinaus kann der Kit gegebenenfalls in separater Form Mittel zum Nachweis von Makrolid-Antibiotikumresistenzen in Mikroorganismen, die zuvor beschriebenen Hybridisierungssonden, und geeignete Puffer enthalten.

Darüber hinaus betrifft die Erfindung die Verwendung des genannten Reagenzienkits und darin die Verwendung eines Oligonukleotids mit einer Nukleotidsequenz, die zu dem 16S rRNA-Bereich von *H.pylori* komplementär ist, der den *E.coli* 16S rRNA Positionen 110 bis 140 (insbesondere 120 bis 137), 740 bis 780 (insbesondere 753 bis 770), 580-610 (insbesondere 585-605) oder 210-245 (insbesondere 219-240) entspricht, zum Spezies-spezifischen Nachweis von *Helicobacter pylori* oder/und *Helicobacter nemestriniae*. Überraschenderweise wurde festgestellt, daß derartige Oligonukleotide, d.h. Nukleinsäuren oder Nukleinsäureanaloga mit einer Länge von vorzugsweise 10 bis 30 Nukleotidbausteinen, bei Verwendung als Amplifikationsprimer oder Hybridisierungssonden eine signifikant höhere Spezifität bezüglich der Erkennung verschiedener *H. pylori-*Isalate als andere Sequenzen aufweisen und darüber hinaus eine zuverlässige Unterscheidung von *H. pylori* und anderen verwandten Spezies erlauben. Vorzugsweise verwendet man ein Oligonukleotid, das die in SEQ ID NO. 5, 6, 7 oder 8 dargestellten Sequenzen oder zumindest einen 10 Nukleotide langen Teilbereich davon enthält. Darüber hinaus betrifft die Erfindung die Verwendung eines Oligonukleotids mit einer Nukleotidsequenz, die zu dem 16S rRNA-Bereich von *H.heilmannii* komplementär ist, der den E.coli 16S rRNA Positionen 580-610 oder/und 640-670 entspricht, zum spezifischen Nachweis von Vertretern der *H.heilmannii-*Gruppe. Vorzugsweise verwendet man ein Oligonukleotid, das die in SEQ ID NO. 9, 10, 11 oder 12 dargestellten Sequenzen oder einen zumindest 10 Nukleotide langen Teilbereich davon enthält.

Vorzugsweise trägt das Oligonukleotid eine Markierungsgruppe. Die Spezies-spezifischen Sonden können im erfindungsgemäßen Verfahren in Kombination mit Hybridisierungssonden zum Nachweis von Makrolid-Antibiotikumresistenzen eingesetzt werden. Außerdem ist bevorzugt, daß in einem Test zwei oder mehr Spezies-spezifische Sonden eingesetzt werden, wobei sichergestellt wird, daß auch weniger biologisch aktive *Helicobacter* Zellen erfaßt werden, die in der Regel eine geringere Anzahl von Ribosomen enthalten.

Noch ein weiterer Gegenstand der Erfindung ist die Verwendung eines Oligonukleotids aus einer bakteriellen 23S rRNA, insbesondere aus einem Bereich, der das Peptidyltransferasezentrum enthält, zum Nachweis von Makrolid-Antibiotikumresistenzen. Vorzugsweise werden Oligonukleotide verwendet, die spezifisch für eine mit Makrolid-Antibiotikumresistenzen assoziierte Mutation der Wildtypsequenz sind und einen Bereich entsprechend einem oder mehreren der Nukleotide 2032, 2057, 2058, 2059, 2503 und 2611 auf der E.coli 23S rRNA umfassen. Besonders bevorzugt verwendet man Oligonukleotide mit der in SEQ ID NO. 1, SEQ ID NO.2 oder SEQ ID NO. 3 dargestellten Sequenz. Diese Makrolid-Antibiotikumresistenz spezifischen Oligonukleotide können gegebenenfalls zusammen mit einem Wildtyp-spezifischen Oligonukleotid verwendet werden, das gegen den gleichen Bereich wie die Resistenz-spezifische Sonde gerichtet ist. Vorzugsweise hat das Wildtyp-spezifische Oligonukleotid die in SEQ ID NO. 4 dargestellte Sequenz.

Somit betrifft die vorliegende Erfindung auch Oligonukleotide, d.h. Nukleinsäuren oder/und Nukleinsäureanaloga, die die in SEQ ID NO.1, 2 oder 3 dargestellte Sequenz oder zumindest einen 10 Nukleotide langen Teilbereich davon enthalten. Gegenstand der Erfindung sind auch Zusammensetzungen, die zwei oder mehrere der genannten Oligonukleotide enthalten. Vorzugsweise tragen ein oder mehrere der Oligonukleotide eine Markierungsgruppe.

Weiterhin wird die Erfindung durch die nachfolgenden Abbildungen und Beispiele erläutert. Es zeigen:
- Abb. 1: eine schematische Darstellung des Peptidyltransferasezentrums in bakterieller 23S rRNA. Es handelt sich um eine lineare Darstellung, in der unterstrichen die Positionen angezeigt werden, an denen bereits Resistenzmutationen gefunden wurden. Gefüllte Kreise stellen die Footprints von Erythromycin dar. Die oberen Zahlen beziehen sich auf die Helix-Nummerierung nach Brimacombe und die unteren Zahlen auf die Nukleotidposition in E.coli nach Brosius.
- SEQ ID NO. 1 - 12: die Nukleotidsequenzen der Sonden ClaR1 (1), ClaR2 (2), ClaR3 (3), ClaWT (4), Hpyl-165-753 (5), 120b (6), Hpyl-16S-585 (7) und Hpyl-16S-219 (8), Hh1 (9), Hh2 (10), Hh3 (11) und Hh4 (12).

### Beispiele

### Beispiel 1

Entwicklung einer *H*.*pylori*- und *H.heilmannii-*spezifischen Sondenkombination für die in situ Hybridisierung zum Nachweis Antibiotikasensitiver *Helicobacter* Bakterien.

Ein Alignment mit 108 annährend vollständigen 16S rRNA Sequenzen von Organismen aus der ∈-Gruppe der Proteobakterien (davon 50 *Helicobacter* Sequenzen und davon wiederum 10 *H.pylori* Sequenzen) wurde zur Ableitung von spezifischen *H.pylori* Sonden herangezogen (Neefs et al., 1993). Insgesamt konnten 4 potentielle Zielsequenzbereiche zugeordnet werden, aus denen verschiedene Sondenprototypen zur weiteren Austestung abgeleitet wurden. Die Austestung der verschiedenen Sondenprototypen in einer in situ Hybridisierung ist ein wichtiger Bestandteil der Sondenentwicklungsstrategie. Es gibt Daten, daß bestimmte Bereiche der ribosomalen RNA nicht für die in situ Hybridisierungsreaktion zur Verfügung stehen. Als Ursache dafür werden stabile Sekundärstrukturen und die Belegung von Sondenbindungsstellen mit ribosomalen Proteinen diskutiert (Amann et al., 1995; Frischer, 1996). Insbesondere müssen bei dieser Austestung solche Sondensequenzen herausgefunden werden, welche die nächsten verwandten Spezies von *H.pylori* wie *H*.*mustelae, H.felis, H.fenneliae, C.coli, C.jejuni* und *W. succinogenes* ausschließen (siehe Tabelle 5). Das im Rahmen der Austestung angewandte in situ Hybridisierungsverfahren ist im nachfolgenden Beispiel 2 im Detail beschrieben.

Schließlich konnte pro Region jeweils ein wirksamer Bereich zur Sondenkonstruktion bestimmt werden, in dem die Sonden Hpyl-16S-753, Hypl-165-120b, Hpyl-16S-585, Hpyl-16S-219 lokalisiert sind. Durch weitere, umfangreiche in situ Bindungsstudien an klinischen Proben haben sich zwei Sondensequenzen als besonders wirksam erwiesen: Hpyl-16S-585 und Hpyl-16S-219. Hpyl-16S-585 ist gegen einen Sequenzbereich der 16S rRNA gerichtet, der nur bei *H*.*pylori* und *H.nemestrinae* vorkommt, nicht aber bei anderen bakteriellen Spezies gefunden wird (Tabelle 3). Die Sonde Hpyl-16S-219 ist gegen eine andere *H*.*pylori* spezifische Region gerichtet. Beide Sonden wurden über einen Aminolinker am 5'-Ende entweder mit dem Fluoreszenzfarbstoff Fluorescein (emittiert grüne Fluoreszenz) oder Cy3 (emittiert rote Fluoreszenz) versehen. Der gleichzeitige Einsatz beider Sonden zum in situ Nachweis von *H.pylori* ist insbesondere wichtig, um metabolisch weniger aktive *H*.*pylori* Zellen zu detektieren. Bakterien mit reduziertem Metabolismus haben in der Regel einen geringeren Gehalt an Ribosomen bzw. rRNA wodurch das Nachweisverfahren an Empfindlichkeit einbüßt, insbesondere dann, wenn nur eine Sonde verwendet wird und diese mit dem vergleichsweise schwach emittierenden Fluorescein-Farbstoff versehen ist.

Weiterhin konnten auch für *H.heilmannii* spezifische Nachweissonden Hh1, Hh2, Hh3 und Hh4 aus der 16S rRNA identifiziert werden (Tabelle 3).

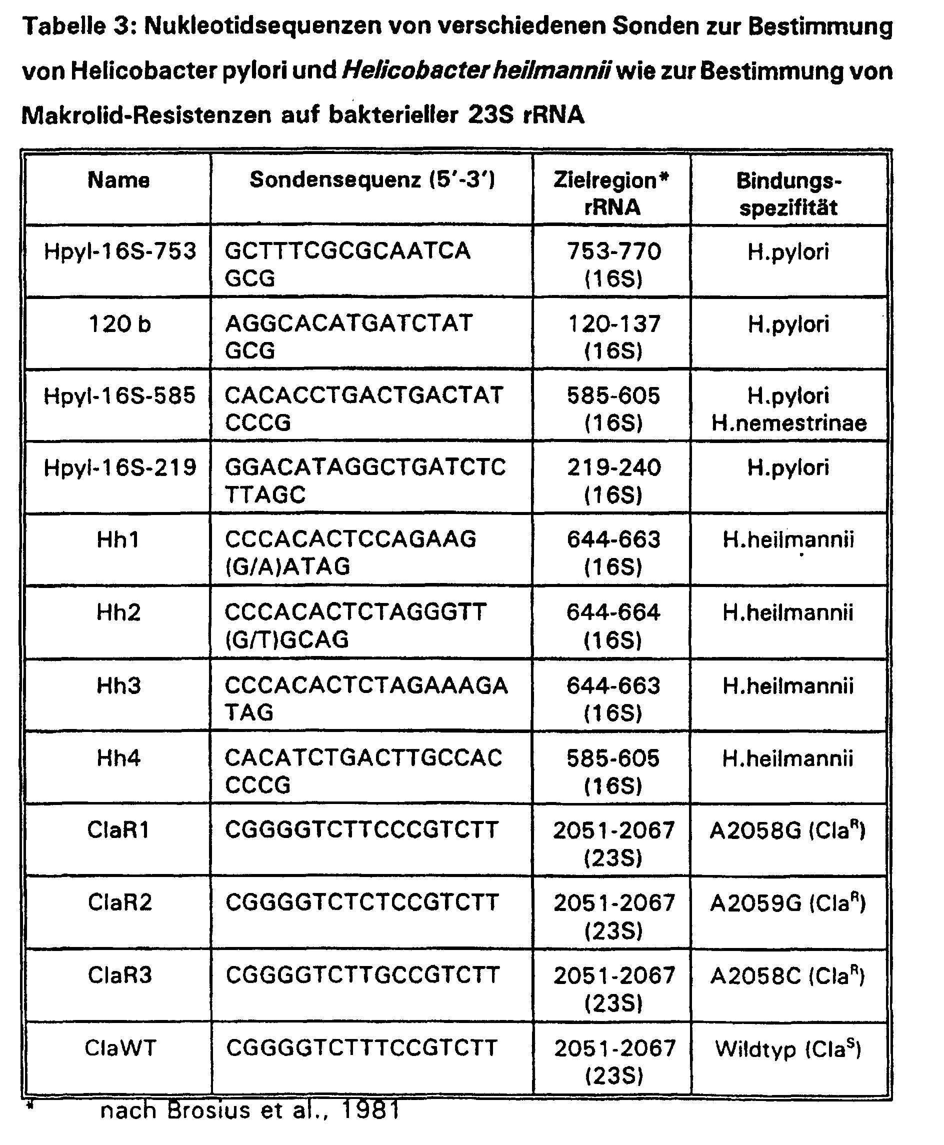

Zum Austesten der Sonden werden verschiedene Referenzzellen wie bei Amann et al. beschrieben mit 3% gepufferter Paraformaldehydlösung fixiert und mittels Lufttrocknung auf Objektträger immobilisiert (Amann et al., 1990).

5 ng der Sonde werden in einem Hybridisierungspuffer ( 0,9 M NaCI, 0,02 M Tris/HCl pH 8,0, 0,01 % SDS, 20% Formamid) bei 46°C für 90 min mit diesen Objektträgern inkubiert. Anschließend wird bei 48°C für 15' gewaschen (0,25 M NaCI, 0,02 M Tris/HCI pH 8,0, 0,01% SDS). Überschüssiger Waschpuffer wird mit PBS von den Objekträgern entfernt und diese zur Verringerung von Ausbleicheffekten in Citifluor AF1 (Citifluor Ltd., London, UK) eingebettet. Die Analyse der Hybridisierung erfolgt mit dem Epifluoreszenzmikroskop (Standardfilter für rote und grüne Fluoreszenz). Bei den angegebenen Bedingungen hybridisierten alle der bisher ausgetesteten *H*. *pylori* Stämme (16/16) mit der Sonde Hpy-16S-753, während Zellen anderer *Helicobacter* Spezies und weitere Referenzstämme (6/6) keine Bindung der Sonde zeigten (Tabelle 4). Dabei handelte es sich um die nächsten verwandten Spezies von *Helicobacter pylori* wie *H*. *mustelae*, *H. felis*, *H*. *fennelliae*, *C*. *coli*, *C*. *jejuni* und *W*. *succinogenes* (Tabelle 4). Die Sonde 120b reagierte mit 11 von 16 Stämmen und zeigte damit eine etwas geringere Spezifität bezüglich der Erkennung verschiedener *H. pylori* Isolate (Tabelle 4).

**Tabelle 4:**

| **Austestung der Spezifität von zwei *H*. *pylori* Sonden** | | |
|---|---|---|
| **Stamm** | **Sonde 120b2 (rot)** | **Hpyl-16S-753 (rot)** |
| *H. pylori* P1 | + + | + + |
| *H. pylori* P79 | + + | + + |
| *H*. *pylori* P79B6.1 | n.d. | n.d. |
| *H*. *pylori* P2 | + + | + + |
| *H*. *pylori* P8 | + + | + + |
| *H. pylori* P12 | - | ++ |
| *H. pylori* P80 | n.d. | n.d. |
| *H. pylori* P80B6.1 | - | + + |
| *H*. *pylori* P21 | + + | + + |
| *H. pylori* P27 | + + | + + |
| *H. pylori* P29 | + + | + + |
| *H. pylori* P31 | + + | + + |
| *H. pylori* P49 | + + | + + |
| *H. pylori* P76 | + + | + + |
| *H*. *pylori* P66 | + + | + + |
| *H. pylori* P92' | - | + + |
| *H. pylori* P106 | - | + + |
| Cl^{r} | - | + + |
| | | |
| *Helicobacter musteleae* NCTC12032 | n.d. | - |
| *Helicobacter felis* ATCC49179 | n.d. | - |
| *Helicobacter fennelliae* | n.d. | - |
| *Campylobacter coli* | n.d. | - |
| *Campylobacter jejuni* | n.d. | - |
| *Wolinella succinogenes* | n.d. | - |
| + +, gute Hybridisierung; -, keine Hybrisisierung | | |

### Beispiel 2

Anwendung rRNA-gerichteter fluoreszenzmarkierter Oligonukleotidsonden zum in situ Nachweis von Clarithromycin-resistenten H.pytori.

Mittlerweile sind drei verschiedene Mutationen in der 23S rRNA beschrieben worden, die Resistenz gegen das Makrolid Clarithromycin vermitteln können (Versalovic et al., 1997). Entsprechend dieser Angaben können Clarithromycin-Resistenzen durch eine A ⇒ G Transition entweder in der Position 2058* (*Nomenklatur n. Brosius) oder 2059* verursacht werden oder durch eine A ⇒ C Transversion an der Position 2058*. Um alle drei resistenzvermittelnden Punktmutationen gleichzeitig nachweisenzu können, wurden drei verschiedene Sondensätze konstruiert, die den genannten Bereich in unterschiedlicher Weise abdecken. In Anbetracht der besonderen Anforderungen einer in situ Hybridisierung wurden die verschiedenen Sondensätze analog zu den *H*.*pylori* spezifischen Sonden hinsichtlich ihrer in situ Bindungseigenschaften ausgetestet (siehe Beispiel 1). Die entscheidenden Auswahlkriterien waren: (1) eindeutiger Nachweis der jeweiligen Punktmutation, (2) gleichzeitiger Nachweis der Punktmutation und der *H*.*pylori* spezifischen rRNA Abschnitte durch gleichzeitigen Einsatz der im Beispiel 1 ausgewiesenen Sonden.

Schließlich konnten für den in situ Nachweis der drei resistenzvermittelnden Mutationen drei wirksame Sonden ermittelt werden: ClaR1, ClaR2 und ClaR3. Die Sonden ClaR1 und ClaR3. Die Sonden ClaR1 und ClaR3 sind komplementär zu der an Position 2058 * veränderten rRNA, während Sonde ClaR2 komplementär zu der an Position 2059* mutierten rRNA ist. Die Sonden wurden über einen Aminolinker am 5'-Ende entweder mit dem Fluoreszenzfarbstoff Fluorescein (emittiert grüne Fluoreszenz) oder Cy3 (emittiert rote Fluoreszenz) versehen. Die Markierung der Sonden mit dem stärker emittierenden Fluoreszenzfarbstoff Cy3 ist von Vorteil, da hierdurch eine höhere Empfindlichkeit des Nachweises sichergestellt ist, insbesondere wenn es sich um metabolisch weniger aktive *H.pylori* Zellen handelt (s.o.).

Mit den oben beschriebenen Sonden wurden geeignete Bedingungen ausgetestet und schließlich an insgesamt 20 Clarithromycin-resistenten und 15 Clarithromycin-sensitiven *H*.*pylori* Stämmen überprüft. Die Spezifität des Resistenznachweises bezüglich *H.pylori* konnte eindeutig demonstriert werden, da die beiden Sonden H.pyl-16S-219 und Hpyl-16S-585 mit allen untersuchten H.pylori-Stämmen reagierten, wobei alle verwandten Helicobacter Arten nicht erfaßt wurden. ClaR1 erkennt die Mutation A2058G (Cla^{R}), ClaR2 erkennt die Mutation A2059G (Cla^{R}) und ClaR3 erkennt die Mutation A2058C (Cla^{R}).

Eine Resistenzbestimmung sollte vorzugsweise mit allen fünf Sonden (ClaR1-3; Hpyl-16S-585; Hpyl-16S-219) durchgeführt werden, da hierdurch eine optimale Aussagekraft erzielt wird (s.u.). Es hat sich in dieser Ausführungsform als vorteilhaft erwiesen, den Nachweis der Punktmutationen in einem kompetitiven Hybridisierungsansatz zu führen und somit die Spezifität dieses Nachweises zu gewährleisten. Dem Sondengemisch wurde diesem Zweck zusätzlich eine Sonde beigemischt, die komplementär zur Wildtypsequenz im Bereich der resistenzvermittelnden Mutationen liegt. Nach entsprechenden Austestungen wurde die Sequenz der Sonde ClaWT ermittelt. Die drei Cy3-markierten Sonden ClaR1, ClaR2 und ClaR3 werden gemeinsam mit einer äquimolaren Menge der unmarkierten ClaWT-Sonde eingesetzt. Um gleichzeitig die Identität der vorliegenden Bakterien anzuzeigen, werden zusätzlich die beiden FLUOS-markierten *H.pylori*-spezifischen Sonden eingesetzt. Entsprechend der dargestellten Anwendungsform können somit folgende Aussagen getroffen werden:
1. Gelbes Fluoreszenz-Signal (Mischfarbe aus rot (Resistenz) und grün (*H.pylori*) *=* Clarithromycin-resistenter *H.pylori*
2. Grünes Fluoreszenz-Signal
   = Clarithromycin-sensitiver *H*.*pylori*
3. Rotes Fluoreszenz-Signal
   = Clarithromycin-resistentes Bakterium unbekannter Art.

Eine 100%ige Spezifität des eingesetzten Sondengemisches wurde unter folgenden Hybridisierungsbedingungen erzielt:
- Hybridisierung:: 90 Minuten bei 46°C in 0,9 M NaCI; 0,02 M Tris/HCI pH 8,0; 0,01% SDS; 20% Formamid.
- Waschschritt:: 15 Minuten bei 48°C in 0,25 M NaCI, 0,02 M Tris/HCI pH 8,0, 0,01 % SDS

**Tabelle 5**

| **Austestung der ausgewiesenen Einzelsonden an verschiedenen bakteriellen Stämmen und an Magenbiopsine (Antrum) durch in situ Hybridisierung** | | | | | | | |
|---|---|---|---|---|---|---|---|
| Probenzusammensetzung | Anzahl¹ | Hypl- | ClaR1 | ClaR2 | ClaR3 | WT | EUB |
| | | 16S-585 | | | | | |
| **A) Bakterielle Stämme** | | | | | | | |
| H.pylori | 35 | 35 | 6 | 12 | 2 | 15 | 35 |
| H.mustelae | 1 | 0 | 0 | 0 | 0 | 1 | 1 |
| H.felis | 1 | 0 | 0 | 0 | 0 | 1 | 1 |
| H.fenneliae | 1 | 0 | 0 | 0 | 0 | 1 | 1 |
| Campylobacter jejuni | 1 | 0 | 0 | 0 | 1 | 0 | 1 |
| C.coli | 1 | 0 | 0 | 0 | 0 | 1 | 1 |
| Wollinella succinogenes | 1 | 0 | 0 | 0 | 0 | 1 | 1 |
| **B) Magenbiopsieproben** | 27² | 17 | 4 | 1 | 0 | 13 | 17 |

| | | | | | | | |
|---|---|---|---|---|---|---|---|
| ¹ Gesamtzahl untersuchter Stämme | | | | | | | |
| ² Gesamtzahl untersuchter Biopsieproben. In einer Biopsieprobe wurden 2 *H.pylori* Stämme nachgewiesen, ein Clarithromycin-resistenter und ein -sensitiver Stamm. | | | | | | | |

### Beispiel 3

In situ Clarithromycin-Resistenzbestimmung von *H*. *pylori* in Gewebeschnitten.

Die *in situ* Resistenzbestimmung von *H. pylori* in Gewebeschnitten wurde zunächst in einem Tiermodell evaluiert. 6 - 8 wöchige C57BI6 Mäuse wurden oral mit 10 Kolonie bildenden Einheiten des *H*. *pylori* Stammes P76 infiziert. 4 Tage nach der Infektion wurde die Maus getötet und der Magen steril entfernt. Der Magen wurde mit dem Skalpell zerkleinert und die Teile für 12 h in einer 3 % Paraformaldehydlösung eingelegt. Es folgten zwei Waschschritte von jeweils einer Stunde in einer gepufferten Salzlösung, z.B. PBS. Anschließend wurde das Material in ein Medium für Gefrierschnitte eingelegt, worauf das ganze bei -70°C eingefroren wurde. Schließlich wurde von dem eingefrorenen Material mit einem Kryomikrotom Gefrierschnitte von jeweils 5-10 µm Dicke angefertigt und auf Polylysin beschichteten Objektträgern fixiert. Die Durchführung der Nachweisreaktion gemäß den beschriebenen Bedingungen erbrachten ein positives Ergebnis, dergestalt, daß sich auf der Krypteninnenseite der Magenprobe deutlich einzelne *H*. *pylori* nachgewiesen werden konnten.

In der Fortsetzung der Untersuchung wurden nunmehr Paraffinschnitte von Magenbiopsaten chronisch infizierter Menschen verwendet, deren mikrobiologischer Hintergrund vorab durch konventionelle Verfahren bestimmt wurde. Die Untersuchung der Gewebeschnitte erfolgte gemäß dem Standardprotokoll, wobei die beiden FLUOS-markierten *H*. *pylori*spezifischen Sonden, die Cy3-markierten Sonden ClaR1, ClaR2 und ClaR3 und die unmarkierte ClaWT-Sonde eingesetzt wurde. Insgesamt wurden 27 Magenbiopsieproben untersucht, wobei in 17 Fällen korrekt, d.h. in Übereinstimmung mit dem konventionellen Verfahren, eine *H*. *pylori* Infektion diagnostiziert werden konnte. In 5 Fällen wurde korrekt eine Clarithromycin-Resistenz nachgewiesen. In einem Fall wurde sogar eine *H*. *pylori* Mischinfektion nachgewiesen, bestehend aus einem Clarithromycin-resistenten und einem Clarithromycin-sensitiven Stamm (siehe Tabelle 5).

In einer anderen Anwendungsform werden die Magenbiopsate nicht fixiert und histologisch aufgearbeitet, sondern in ein spezielles Transport- bzw. Presumptive-Medium überführt, das ein langes Überleben des Keims gewährleistet und gegebenenfalls das Vorhandensein von *H*. *pylori* in der Probe anzeigt (siehe Beispiel 5). Entnimmt man dem Transportmedium nach unterschiedlich langer Aufbewahrungszeit Proben und untersucht diese mit dem beschriebenen Verfahren, so können die Bakterien überraschender Weise noch nach 7 Tagen eindeutig charakterisiert werden. Besonders überraschend war das Ergebnis, daß die Probe hierzu nicht unbedingt gekühlt (4°C) aufbewahrt werden muß.

**Tabelle 6**

| **Kultivierungs- und Hybridisierungsergebnisse von *H*. *pylori* Hpci 001- Tübingen nach unterschiedlich langem Transport in CREATOGEN- Transportmedium** | | | | |
|---|---|---|---|---|
| | Aufbewahrung bei Raumtemperatur | | Aufbewahrung bei 4°C | |
| H.pylori Nachweis aus Transportmedien | Konventionelle Kultivierung | In situ Hybridisierung | Konventionelle Kultivierung | In situ Hybridisierung |
| 6h | + | + | + | + |
| 1 Tag | + | + | + | + |
| 2 Tage | - | + | + | + |
| 3 Tage | - | + | + | + |
| 4 Tage | - | + | + | + |
| 7 Tage | - | + | - | + |
| - kein Nachweis + Nachweis erbracht | | | | |

### Beispiel 4

Vergleich der 23S-rRNA Sequenz verschiedener medizinisch bedeutsamer Bakterien im Bereich der Clarithromycin-Resistenz-Region.

Mit Hilfe des Computerprogrammes ARB (Ludwig et al., TU München) wurde die 23S-rDNA von 40 Gram-positiven und Gram-negativen Bakterien mit der Makrolid-resistenzvermittelnden Region von *H*. *pylori* verglichen (Tabelle 6). Die Ergebnisse zeigen bei allen untersuchten Bakterien eine starke Konservierung der Primärstruktur dieser speziellen Region der ribosomalen RNA in der die Resistenz gegen Makrolid-Antibiotika determiniert wird. Allerdings scheint die Bindungsspezifität der ausgewiesenen Sonden ClaR1, ClaR2 und ClaR3 für *H*. *pylori* erfüllt zu sein. Da es sich bei den 40 Sequenzen aus der Datenbank wahrscheinlich um die Sequenzen Clarithromycin-sensitiver Bakterien handelt und man davon ausgehen kann, daß die Clarithromycin-Resistenz bei diesen Bakterien, wie bei H. pylori, ebenfalls durch eine Mutation in der Position 2058/2059 der 23S-rDNA (n. Brosius) determiniert wird, kann das beschriebene Verfahren zur in situ Bestimmung von Makrolid-Resistenzen auch bei diesen Bakterien angewandt werden. Es muß lediglich zur eindeutigen Zuordnung des entsprechenden pathogenen Keims, der mit der Krankheitsbildung verknüpft ist, eine Spezies-spezifische Sonde abgeleitet werden und in der beschriebenen Weise mit dem Sondengemisch zur Identifizierung der resistenzvermittelnden Mutation kombiniert werden.

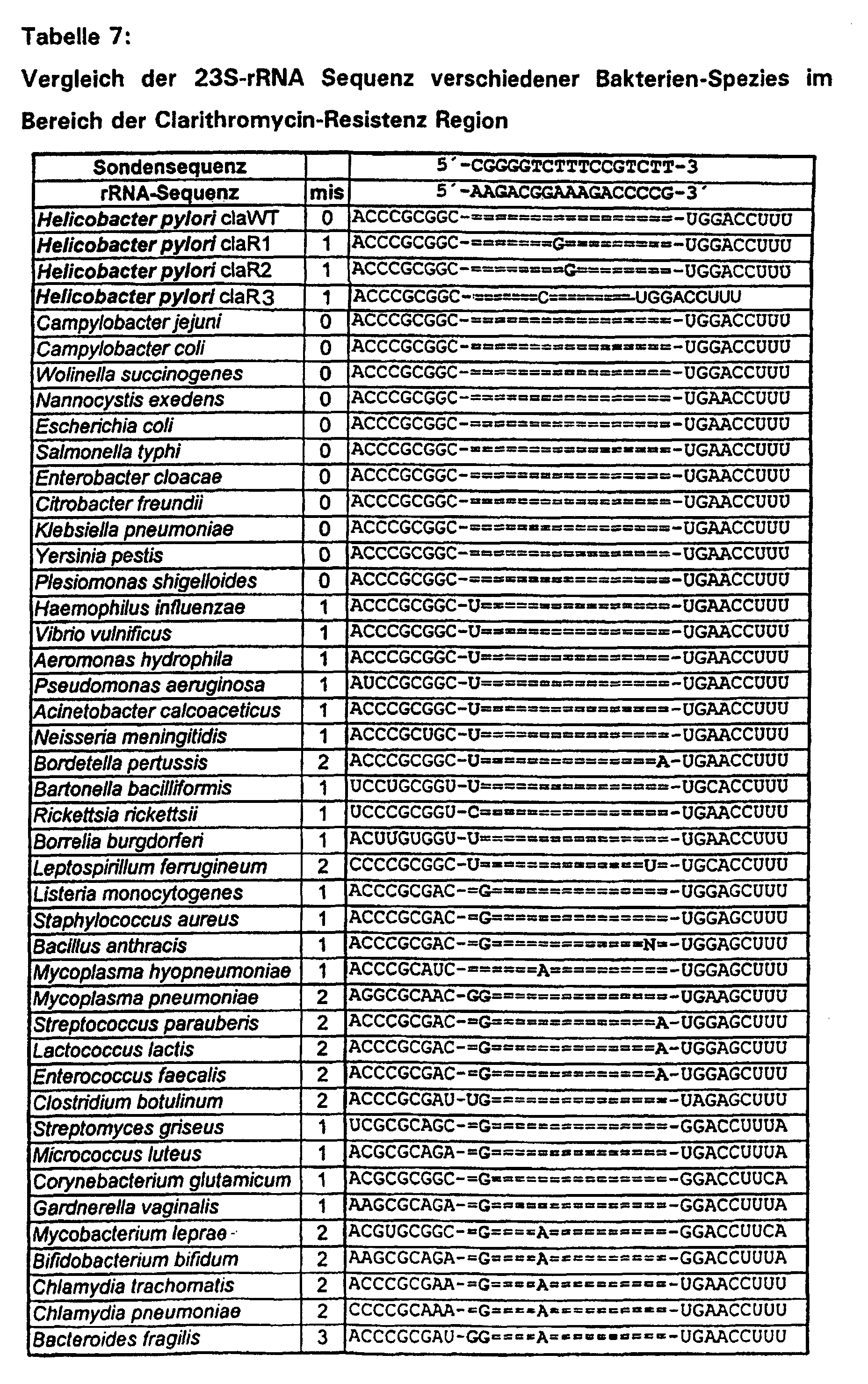

mis, Anzahl der mismatches zur rRNA Sequenz in dieser Region. Die Startposition der Sondensequenz in der 23S-rRNA der verschiedenen Spezies entspricht Position 2051 in E.coli (Brosius et al., 1981), N, entspricht A, C, G oder T. = identisch zur rRNA-Sequenz.

### Beispiel 5

### Entwicklung eines Presumptive-Mediums für bakterielle Gastritis mit kombiniertem Urease-Nachweis speziell für Helicobacter pylori

Im Vordergrund steht die Entwicklung eines Presumptive-Mediums, das die Konservierung lebensfähiger *Helicobacter pylori* aus Magenbiopsien über einen Zeitraum von 5 Tagen, mindestens jedoch über 48 Stunden, ermöglichen soll. Darüber hinaus soll die Begleitflora, sofern vorhanden, erhalten werden, um eine vollständige mikrobiologische Befunderhebung sicherzustellen. Das Wachstum der Keime muß eingeschränkt werden, um die anteilige Zusammensetzung der Keime in der Biopsie möglichst unverändert zu halten. Das Presumptive-Medium besteht im wesentlichen aus einer gepufferten Nährlösung, die bevorzugt in einer halbfesten organischen Matrix vorliegt, wie z.B. (0,2 - 1,5 %) Agar oder (mind. 15 %) Gelatine. Die Nährlösung beinhaltet eine Stickstoffquelle und weitere essentielle Komponenten, welche die Stabilität von Helicobacter verbessern. Die Stickstoffquelle nimmt 0,5 bis 5 % des Presumptive-Mediums ein. Sie besteht aus chemischen oder proteolytischen Aufschlüssen von Proteinen mikrobieller, tierischer oder pflanzlicher Herkunft, wie z.B.: Peptone, Tryptone oder Casitone oder Gemische von diesen. Besonders bevorzugt sind Medien wie Schivo-Medium® oder Medien auf der Basis von "brainheart infusion" (BHI). Weitere bevorzugte Bestandteile sind Hefe-Extrakt (z.B. 0,01 %), Serumproteine, wie Pferdeserum oder foetales Kälberserum oder Rinderserumalbumin oder definierte, organische Substanzen, wie (2,6)Dimethyl)-beta-cyclodextrin und Cholesterol. Die Serumproteine sollten 1 - 10 % des Presumptive-Mediums einnehmen, die organischen Substanzen 0,01 - 0,2 %. Außerdem wird dem Presumptive-Medium reduziertes Cystein oder Thioglykolat zugemischt und/oder Sauerstoff-abweisende Zusätze beigefügt. Alle Komponenten liegen in einer gepufferten wäßrigen Lösung vor, deren bevorzugter pH-Wert zwischen 5,5 und 6,5 liegt. Die Biopsieprobe wird in das untere Drittel der halbfesten Matrix eingebracht, wodurch das Eindiffundieren von Luftsauerstoff verhindert wird, was die Überlebensbedingungen des Keims verbessert.

Die Verknüpfung des Presumptive-Mediums mit einem Urease-Nachweistest ist neu. Sie bietet dem Arzt den Vorteil, daß dieser rasch eine mögliche *Helicobacter* Infektion angezeigt bekommt und direkt weitere Maßnahmen einleiten kann. Das Presumptive-Medium wird zu diesem Zweck zusätzlich mit (0,5 - 5 %) Harnstoff und (0,001 - 0,01 %) Phenolrot oder anderen pH-Indikatoren, wie Bromocresolpurpur, versetzt. Die Umsetzung des Harnstoffs durch die Urease führt u.a. zur Entstehung von Ammoniak, welches den pH-Wert ins Basische verändert und z.B. den pH-Indikator Phenolrot von gelblich in rot bis violett umschlagen läßt. Die Geschwindigkeit des Farbumschlags, die Farbintensität und die Farbgebung korrelieren direkt mit der Menge Urease-produzierender Bakterien. Die Bestandteile des kombinierten Presumptive-Mediums sind so eingestellt, daß eine hohe Empfindlichkeit erreicht wird (500 - 2000 Keime/Presumptive-Medium) und eine Autolyse der Keime unterdrückt ist. Alternativ kann dem Presumptive-Medium ein synthetisches Urease-Substrat beigemischt werden, dessen Umsetzung einen Farbumschlag bewirkt.

Vergleichsuntersuchungen belegen, daß andere, routinemäßig angewandte Urease-Nachweisverfahren zu einer raschen Lyse der Keime führen. Dies ist von größter Bedeutung, da neuere Untersuchungen belegen, daß in den Biopsie-Proben oftmals andere Urease-produzierenden Keime, wie z.B. *Proteus mirabilis*, *Klebsiella oxytoca* und *Pseudomonas aeruginosa* vorzufinden sind. Im Unterschied zu *Helicobacter* sezernieren diese Keime keine Urease, sondern das Enzym liegt im Zellinnern vor. In eigenen Untersuchungen wurde festgestellt, daß es in gängigen Urease-Nachweisverfahren, wie dem CLO-Test, zur Lyse der Bakterien kommt, wobei die intrazelluläre Urease freigesetzt wird, die das nun verfügbare Substrat sofort umsetzt. Eine vorläufige Untersuchung von 32 Patienten mit akuter Gastritis hat ergeben, daß mit herkömmlichen Verfahren nur aus 34 % der mikrobiologisch untersuchten Biopsien ein *Helicobacter* isoliert werden konnte. Dabei kann nicht ausgeschlossen werden, daß die *Helicobacter*-freien Biopsien mit Stämmen besiedelt waren, die sich sehr schlecht anzüchten lassen. Bedeutsam ist allerdings, daß ein Großteil (> 50 %) der *Helicobacter*-freien Biopsien andere Urease-produzierende Keime enthielten, die von den gängigen Urease-Nachweisverfahren angezeigt wurden. Das kombinierte Transport- und Indikatormedium verzögert bzw. verhindert eine Lyse dieser Bakterien und garantiert somit einen *Helicobacter*-spezifischen Urease-Nachweis.

**Tabelle 8:**

| Ureaseproduzierender Keim¹ | Farbumschlag des Presumptive-Mediums² | |
|---|---|---|
| | 5 Stunden | 24 Stunden |
| Helicobacter pylori | + + (< 10 Minuten) | + + + |
| Proteus mirabilis | +/- (> 3 Stunden) | + + |
| Klebsiella oxytoca | - | - |
| Pseudomonas aeruginosa | - | - |

| | | |
|---|---|---|
| ¹ Für den Test wurde jeweils eine komplette Bakterienkolonie eingesetzt. | | |
| ² Das Presumptive-Medium wurde bei Raumtemperatur (21 - 25°C) inkubiert und visuell ausgewertet. | | |

### Beispiel 6

Antibiotika-Empfindlichkeitstestung Clarithromycin-resistenter *H*. *pylori* Sämme mittels *in situ* Nachweis durch rRNA-gerichtete fluoreszenzmarkierte Sonden.

Aufgrund der Beobachtung von Versalovic et al. (1997), daß die verschiedenen resistenzvermittelnden Punktmutationen auf der 23S rRNA mit der Empfindlichkeit des Keims gegenüber Clarithromycin korrelieren, ergibt sich anhand der vorliegenden, neuen Resultate die Möglichkeit durch den Einsatz der einzelnen Sonden (ClaR1 - 3) eine direkte Antibiotika-Empfindlichkeitstestung durchzuführen.

So führen Mutationen in der Position A2058G zu signifikant höheren Resistenzwerten als Mutationen in der Position A2059G. Wie schon gezeigt, wird die Mutation in der Position A2058G mit der Sonde ClaR1 spezifisch nachgewiesen. In einem Optimierungsprozeß wurde der hochspezifische Nachweis der A2058G Punktmutation durch ein Gemisch bestehend aus der Cy3-markierten ClaR1-Sonde und den unmarkierten Sonden ClaWT und ClaR3 erzielt.

In ähnlicher Weise wurde ein funktionierendes Sondengemisch zum spezifischen Nachweis der A2059G Punktmutation entwickelt, bestehend aus der Cy3-markierten ClaR2-Sonde und der unmarkierten ClaWT-Sonde, Zum Nachweis der Punktmutation A2058C wird die Cy3-markierte Sonden ClaR3 verwendet in Kombination mit der unmarkierten Sonde ClaWT.

Die Hybridisierung wird im Beispiel 2 genannten Puffer durchgeführt. Soll die Antibiotika-Empfindlichkeitstestung mit der Identifizierung von *H. pylori* gekoppelt werden, so werden den genannten Sondenkombinationen noch die FLUOS-markierten Oligonukleotide Hpyl-16S-585-FLUOS und Hpyl-16S-219-FLUOS zugegeben.

**Tabelle 8:**

| Evaluierung der MHK-Wert Bestimmung durch *in situ* Hybridisierung mit konventionell charakterisierten *H. pylori* Isolaten. | | | | | | |
|---|---|---|---|---|---|---|
| Spezies | Stamm | ClaR1 | ClaR2 | ClaR3 | Hpyl-16S-585 | MHK-Wert (mg/l) |
| *H. pylori* | MPS1726 | + | - | - | + | >256 |
| | MPS1765 | + | - | - | + | >256 |
| | MC141 | + | - | - | + | > 256 |
| | MPS3286 | - | - | + | + | > 256 |
| | MPS3137 | - | + | - | + | 96 |
| | FD591 | - | + | - | + | 16/32 |
| | KJ472 | - | + | - | + | 16 |
| | HH531 | - | + | - | + | 48 |
| | MC028 | - | + | - | + | 24 |
| | MC132 | - | + | - | + | 12 |
| - Kein Fluoreszenz-Signal nachweisbar. | | | | | | |
| + Nachweis eines eindeutigen Fluoreszenz-Signals | | | | | | |

### Beispiel 7

Das Gesamtverfahren der Resistenzbestimmung setzt sich aus zwei aufeinanderfolgenden Teilschritten zusammen, der Probengewinnung (A) und der Nachweisreaktion (B).

Die Probengewinnung umfaßt im wesentlichen die gezielte Gewinnung von biologischen Material in dem sich der krankheitsverursachende Keim angesiedelt hat. Das gewonnene biologische Material kann dann direkt der Nachweisreaktion zugeführt, durch bekannte Verfahren fixiert oder in ein spezielles Presumptive-Medium (siehe Beispiel 5) überführt werden.

Die Nachweisreaktion (*in situ* Hybridisierung) erfordert im wesentlichen drei Arbeitsschritte, (1) die Immobilisierung der Probe auf einem Träger, (2) die Permeabilisierung der Probe und (3) die eigentliche Hybridisierungsreaktion sowie deren Auswertung.

Im Falle einer mikroskopischen Untersuchung wird die Probe auf einen Objektträger immobilisiert, der in der Regel aus Glas besteht. Es können auch andere Trägermaterialien verwendet werden, z. B. Mikrotiterplatten oder Folien oder Siliciumplatten, in Abhängigkeit der gewählten Untersuchungsform. In der Regel wird die Probe in Form eines Abstrichs auf den Objektträger aufgetragen. Bei Gewebeproben werden bevorzugt Schnitte verwendet. Schließlich wird die Probe über eine Lufttrocknung auf dem Objekträger immobilisiert (Amann et el., 1990), wobei die Fixierung der Probe durch eine Vorbehandlung des Objektträgers, z.B. mit Polylysin oder gar spezifische Rezeptormoleküle, wie Antikörper, verbessert werden kann.

Durch den Permeabilisierungsschritt wird die Probe, insbesondere die in der Probe befindlichen Bakterien für die Sonden durchgängig gemacht. Das Verfahren ist so ausgerichtet, daß insbesondere die Bakterienhülle durchgängig gemacht wird wobei die innere Struktur weitgehend erhalten bleibt. Zur Permeabilisierung von Gram-negativen Bakterien reicht in der Regel ein konventionell durchgeführtes Fixierungsverfahren aus. Die Permeabilisierung Gram-positver Bakterien erfordert zusätzliche Maßnahmen,, z.B. die Verwendung von Komponenten wie etwa organischen Lösungsmitteln, wie z.B. Toluol, Xylol, Aceton, Ethanol, etc., Detergentien, wie z.B. SDS, Nonidet, etc., und Zellwand auflösenden Enzymen, wie z.B. Lysozym, Lysostaphin, Mutanolysin, etc.

Die Hybridisierungsreaktion wird in zwei Schritten durchgeführt, (a) die Inkubation der permeabilisierten Probe mit der markierten Sonde bzw. dem Sondengemisch und (b) ein nachfolgender Waschschritt zum Entfernen unspezifisch gebundener Sonden mit anschließender Auswertung. Die Inkubation der Sonden (1 - 1000 ng) erfolgt in einer wässrigen Lösung für mehrere Stunden bei einer Temperatur, die sich aus der Formel zur Berechnung der Dissoziationstemperatur von RNA-DNA Hybriden herleiten läßt (Lathe, 1985 & Wahl, 1987):Td = 81,5 + 16,6 lg[Na⁺] + 0.4(%GC) - 820/n - 0,5(%FA); n = Länge des Oligonukleotids; FA = Formamid. Die wesentlichen Bestandteile der wässrigen Lösung sind chaotrope Substanzen, wie Salze oder/und Formamid, mit denen eine spezifische Bindung der Sonden an die komplementären Zielsequenzen auf der rRNA erreicht wird und ein Detergenz zur Unterdrückung unspezifischer Bindungen der Sonden z.B. an Proteine. Die Lösung zum Herauswaschen der unspezifisch gebundenen Sonden ist prinzipiell ähnlich zusammengesetzt, wobei die Sondenbindung beeinflussenden Bestandteile in niedrigeren Konzentrationen eingesetzt werden und/oder die Temperatur entsprechend erhöht wird. Schließlich wird der überschüssige Waschpuffer wird mit einer gepufferten Salzlösung, z.B. PBS von den Objekträgern entfernt und der Probenbereich in Citifluor AF1 (Citifluor Ltd., London, UK) eingebettet, um die Ausbleichung der fluoreszierenden Sonden bei der Untersuchung zu verringern. Das Ergebnis der Nachweisreaktion wird mit Hilfe eines Fluoreszenzmikroskops abgelesen (Standardfilter für rote und grüne Fluoreszenz oder/und Mischfilter für den gleichzeitigen Nachweis von roter und grüner Fluoreszenz).

### Beispiel 8

Es gibt Beobachtungen, daß *H*. *pylori* unter unvorteilhaften Lebensbedingungen seine Morphologie ändert und sich von der bekannten Stäbchenform in eine kokkoidale Form umwandelt. Von dieser kokkoiden Form ist bekannt, daß sie nicht mehr kultivierbar ist und somit klassischen, mikrobiologischen Untersuchungen nicht zugänglich ist. Sollte es sich bei diesen Formen tatsächlich um Überdauerungsstadien von *H. pylori* handeln, müssen diese unbedingt einer Diagnose zugeführt werden können.

In einer Versuchsserie wurde überprüft, in wie weit die kokkoiden Formen für das beschriebene Nachweisverfahren zur Bestimmung der Antibiotika-Resistenz verfügbar sind. Ein Clarithromycin-resistenter *H*. *pylori* Stamm wurde in seine kokkoide Form überführt, in dem er für eine Woche bei 4°C in destilliertem Wasser aufbewahrt wurde. Nach dieser Zeitspanne waren alle stäbchenförmigen *H*. *pylori* Zellen vollständig zu kokkoiden Formen transformiert, die auf gängigen Kulturmedien nicht mehr anwuchsen. Diese kokkoiden Zellen wurden dem beschriebenen Nachweisverfahren mit dem bekannten Sondengemisch unterworfen. Tatsächlich gelang es die kokkoide Form als *H*. *pylori* zu identifizieren und, was besonders wichtig ist, die Clarithromycin-Resistenz wurde ebenfalls nachgewiesen. Mittlerweile ist es sogar gelungen, in humanen Gewebebiopsien diese Formen über das beschriebene Verfahren nachzuweisen.

Hiermit steht zum ersten Mal ein Nachweisverfahren für die Bestimmung einer Antibiotika-Resistenz bei kokkoiden Helicobacter zur Verfügung. Darüber hinaus war nicht zu erwarten, daß dieses Verfahren bei kokkoiden *H. pylori* funktioniert. In zahlreichen Arbeiten wurde berichtet, daß die kokkoide Form einen stark reduzierten rRNA Gehalt hat und diese zudem degradiert ist (Donelli *et al*., 1998; Narikawa *et al*., 1997). Unsere Untersuchungen belegen, daß die in H₂O induzierte kokkoide Form einen zum Nachweis ausreichenden rRNA-Gehalt aufweist und sehr gut durch eine, auf die rRNA ausgerichtete *in situ* Hybrisierung charakterisiert werden kann.

Die Untersuchung kokkoider Formen gewinnt insbesondere bei der Resistenzbestimmung an Bedeutung. Es ist bekannt, daß sich kokkoide Formen bevorzugt bei sublethalen Antibiotikakonzentrationen ausbilden und somit diese Formen in einem möglichen Zusammenhang beim Versagen der Antibiotikatherapie stehen. Darüber hinaus ist es wahrscheinlich, daß diese Formen in hohen Konzentrationen im Stuhl infizierter Menschen vorkommen und somit über das beschriebene Verfahren einfach und rasch, d.h. ohne gastroendoskopische Entnahme einer Biopsieprobe, eine akute *H. pylori* Infektion mit einem Clarithromycin-resistenten Keim nachgewiesen werden kann.

### Literaturverzeichnis:

**Amann, R. I.,L. Krumholz and D. Stahl.** 1990. Fluorescent oligonucleotide probing of whole cells for determinative, phylogenetic and environmental studies in microbiology. J. Bacteriol. **172**:762-770.

**Beimfohr, C., A. Krause, R. Amann, W. Ludwig and K.-H. Schleifer.** 1993. In situ identification of Lactococci, Enterococci and Streptococci. System. Appl. Microbiol. **16:**450-456.

**Berry, V., K. Jennings and G. Woodnutt.** (1995) Bactericidal and morphological effects of amoxicillin on Helicobacter pylori. Antimicrob Agents Chemother **39:** 1859-1861.

**Bizzozero, G.** (1893) Ueber die schlauchformigen drusen des magendarmkanals und die bezeihungen ihres epithels zu dem oberflachenepithel der schleimhaut. *Arch Mikr Anast* **42:** 82

**Bode, G., F. Mauch and P. Malfertheiner.** (1993) The coccoid forms of Helicobacter pylori. Criteria for their viability. Epidemiol Infect **111**: 483-490.

**Brimacombe, R. B., B. Greuer, P. Mitchell, M. Osswald, J. Rinke-Appel, D. Schüler, and K. Stade.** 1990. Three dimensional structure and function of *E*. *coli* 16S and 23S rRNA as studied by crosslinking techniques, p. 93-106. In E. Hill, A. Dahlberg, R A. Garrett, P. B. Moore, D. Schlessinger, and J. R. Warner (ed.) The ribosome, structure function, and evolution. American Society of Microbiology, Washington, D. C.

**Brosius, J., T. J. Dull, D. D. Sleeter and H. F. Noller.** 1981. Gene organization and primary structure of a ribosomal RNA operon from *Escherichia coli*. *J.* Mol. Biol. **148:**107-127.

**Buckley, M.J., Xia, H.X., Hyde, D.M., Keane, C.T. and O'Morain, C.A.** (1997) Metronidazole resistance reduces efficacy of triple therapy and leads to secondary clarithromycin resistance. *Dig Dis Sci* **42:** 2111-2115.

**Cao, J., Z.Q Li, K. Borch, F. Petersson and S. Mardh.** (1997) Detection of spiral and coccoid forms of Helicobacter pylori using a murine monoclonal antibody. Clin Chim Acta **267:** 183-196.

**Cellini, L., N. Allocati, D. Angelucci, T. lezzi, E. Di Campli, L. Marzio and B. Dainelli.** (1994) Coccoid Helicobacter pylori not culturable in vitro reverts in mice. Microbiol Immunol **38:** 843-850.

**Chan, W.Y., P.K. Hui, K.M. Leung, J. Chow, F. Kwok and C.S. Ng.** (1994) Coccoid forms of Helicobacter pylori in the human stomach. Am J Clin Pathol **102:** 503-507.

**DeLong, E. F., G. S. Wickham, and N. R. Pace.** 1989. Phylogenetic stains: ribosomal RNA-based probes for the identification of single microbial cells. Science. **243**:1360-1363.

**Dent J.C., C.A.M. McNulty, J.C. Uff, S.P. Wilkinson, and M.W.L. Gear.** 1987. Spiral organism in the gastric antrum, Lancet ii:96.

**Desroches, J.J., Lahaie, R.G, Picard, M., Morais, J., Dumont, A., Gaudreau, C., Picard, D., Chartrand, R.,** Methodological validation and clinical usefulness of carbo-14-urea breath test for documentation of presence and eradication of *Helicobacter pylori* infection. J. Nucl. Med. 1997 38(7) 1141-1145.

**Donelli, G., P. Matarrese, C. Fiorentini, B. Dainelli, T. Taraborelli, E. Di Campli, S. Di Bartolomeo and L. Cellini.** (1998) The effect of oxygen on the growth and cell morphology of Helicobacter pylori. FEMS Microbiol Lett **168:** 9-15.

**Eaton, K.A., C.E. Catrenich, K.M. Makin, and S. Krakowka.** (1995) Virulence of coccoid and bacillary forms of Helicobacter pylori in gnotobiotic piglets. JID **171:** 459-462.

**Forman, D., Coleman, M., Debacker, G., Eider, J., Moller, H., Damotta, L.C., Roy, P., Abid, L., Tjonneland, A., Boeing, H., Haubrich, T., Wahrendorf, J., Manousos, O., Tulinius, H., Ogmundsdottir, H., Palli, D., Cipriani, F., Fukao, A., Tsugane, S., Miyajima, Y. et al.** (1993) An international association between *Helicobacter pylori* infection and gastric cancer. *Lancet* **341:** 1359-1362.

**Forsgren J., A. Samuelson, A. Ahlin, J. Jonasson, B. Rynnel-Dagöö, and Alf Lindberg.** *Haemophilus influenzae* resides and multiplies intracellularly in human adenoid tissue as demonstrated by in situ hybridization and bacterial viability assay. Infect. Immun. 1994. **62:** 673-679.

**Frischer, M.E., Floriania P.J., and Nierzwicki-Bauer,** 1996. Differential sensitivity of 16S rRNA targeted oligonucleotide probes ... Can. J. Microbiol. **42:**1061-1071.

**Giovannoni, S. J., E. F. De Long, G. J. Olsen, and N. R. Pace.** 1988. Phylogenetic group-specific oligonucleotide probes for identification of single microbial cells. J. Bacteriol. **170:**720-726.

**Goodwin, C.S., Armstrong, J.A., Chilvers, T., Peters, M., Collins, M.D., Sly, L., McConnell, W. and Harper, W.E.S.** (1989) Transfer of *Campylobacter pylori* and *Campylobacter mustelae* to *Helicobacter* gen. nov. as *Helicobacter pylori* comb. nov. and *Helicobacter mustelae* comb. nov., respectively. *Int J Syst Bact* **39:** 397-405.

**Graham, D.Y.** (1995) Clarithromycin for treatment of *Helicobacter pylori* infections. *Eur J Gastroenterol Hepatol* **7 Suppl** 1: S55-8.

**Hentschel, E., Nemec, H., Schutze, K., Hirschl, A., Dragosics, B., Brandstatter, G and Taufer, M.** (1991) Duodenal ulcer recurrence and *Helicobacter pylori* [letter; comment]. *Lancet* **338:** 569.

**Holck. S., et al.,** 1997. APMIS 105; 746-756.

**Isaacson, P.G. and Spencer, J.** (1993) Is gastric lymphoma an infectious disease? *Hum Pathol* **24:** 569-570.

**Jablonowski, H., Fatkenheuer, G., Youle, M., Newell, T., Lines, S. and Craft, J.C.** (1997) Ancillary benefits of *Mycobacterium avium*-*intracellulare* complex prophylaxis with clarithromycin in HIV-infected patients. *Drugs* **54 Suppl 2:** 16-22.

**Khin, M.M., M. Ringner, P. Aleljung, T. Wadström and B. Ho.** (1996) Binding of human plasminogen and lactoferrin by Helicobacter pylori coccoid forms. J Med Microbiol **45:** 433-439.

**Kusters, J.G., M.M. Gerrits, J.A. Van Strijp and C.M. Vandenbroucke-Grauls..** (1997) Coccoid forms of Helicobacter pylori are the morphologic manifestation of cell death. Infect Immun **65:** 3672-3679.

**Langendijk, P. S., F. Schut, G. J. Jansen, G. C. Raangs, G. R. Kamphuis, M. H. F. Wilkinson, and G. W. Welling.** 1995. Quantitative fluorescence in situ hybridization of Bifidobacterium spp. with genus-specific 16S rRNA-targeted probes and its application in fecal samples. Appl. Environ. Microbiol. **61:** 3069-3075.

**Lathe, R.** (1985) Synthetic oligonucleotide probes deduced from amino acid sequence data. Theoretical and practical considerations. J.Mol.Biol. **183**:1-12.

**Lischewski A., R. I. Amann, D. Harmsen, H. Merkert, J. Hacker, and J. Morschhäuser.** 1996. Specific detection of *Candida albicans* and *Candida tropicalis* by fluorescent in situ hybridization with an 18S rRNA-targeted oligonucleotide probe. Microbiology. **142:** 2731-2740.

**Lischewski A., M. Kretschmar, H. Hof, R. Amann, J. Hacker, and J. Morschhäuser.** 1997. Detection and Identification of Candida Species in experimantally infected tissue and human blood by rRNA-specific fluorescent in situ hybridization. J. Clin. Microbiol. **35:** 2943-2948.

**Lucier, T.S., K. Heitzman, S.-K. Liu, and P.-C. Hu.** 1995. Transition mutation in the 23S rRNA of Erythromycin-resistent isolates of Mycoplasma pneumoniae. Antimicrob.Agents Chemother. **39:** 2770-2773.

**Malfertheiner, P.** (1994) *Helicobacter pylori* - *Von der Grundlage zur Therapie*. Georg Thieme Verlag, Stuttgart, New York.

**Marshall, B.J., Royce, H., Annear, D.I., Goodwin, C.S., Pearman, J.W., Warren, J.R. and Armstrong, J.A.** (1984) Original isolation of *Campylobacter pyloridis* from human gastric mucosa. *Microbios Lett* **25:** 83-88.

**Meier, A., P. Kirschner, B. Springer, V. A. Steingrube, B. A. Brown, R. J. Wallace, and E. Boettger.** 1994. Identification of mutations in the 23S ribosomal RNA gene of clarithromycin resistant *Mycobacterium intracellulare.* Antimicrob. Agents Chemother. **38:**381-384

**Morotomi, M., Hoshina, S., Green, P.** et al., 1989, J. Clin. Microbiol. 27(12):2652-2655.

**Narikawa, S., S. Kawai, H. Aoshima, O. Kawamata, R. Kawaguchi, K. Hikiji, M. Kato, S. lino and Y. Mizushima.** (1997) Comparison of the nucleic acids of helical and coccoid forms of Helicobacter pylori. Clin Diagn Lab Immunol **4** 285-290.

**Neefs J-M., Y. Van de Peer, P. De Rijk, S. Chapelle, and R. De Wachter,** 1993. Compilation of small ribosomal subunit RNA structure. Nuc. Acids Res. **21**:3025-3049.

**Noller, H. F., V. Hoffarth, and L. Zimniak.** 1992. Unusual resistance of peptidyl transferase to protein extractzion procedures. Science. **256**:1416-1419.

**Nordentoft, S., H. Christensen, and H. C. Wegener.** 1997. Evaluation of a fluorescence-labelled Oligonucleotide probe targeting 23S rRNA for in situ detection of Salmonella Serovars in Paraffin-Embedded tissue sections and their rapid identification in bacterial smears. J. Clin. Microbiol. **35:** 2642-2648.

**Palu, G., S. Valisena, M. F. Barile, and G. A. Meloni. 1989.** Mechanism of Macrolide resistance in Ureaplasma urealyticum: a study on collection and clinical strains. Eur. J. Epidemiol. **5**:146-153.

**Ramsing, N. B., M. Kühl, and B. B. Jorgensen.** 1993. Distribution of sulfatereducing bacteria, O₂, and H2S in photosynthetic biofilms determined by olibgonucleotides probes and microelectrodes. Appl. Environ. Microbiol. **59:** 3840-3849.

**Ross et al.** 1997. Clinical resistance to erythromycin and clindamycin in cutaneous propionibacteria isolated from acne patients is associated with mutations in 23S rRNA. Antimicrob. Agents Chemother. **41:** 1162-1165.

**Sambrook, J., E.F. Fritsch, T. Maniatis.** (1989). Molecular cloning: a laboratory manual. Cold Spring Harbor Laboratory Press, Cold Spring Harbor, New York.

**Schaechter, O., O. Maaloe, and N. O. Kjeldgaard.** 1958. Dependence on medium and temperature of cell size and chemical composition durin balanced growth of Salmonella typhimurium. J. Gen. Microbiol. 19:592-606.

**Schwarz, E., Plum, G., Hasbach, H., Eidt, S., Schrappe, M., Kruis, W.:** Molecular biology in diagnosis and epidemiology of *Helicobacter pylori:* PCR for the detection and AP-PCR for characterization of patient isolates. Zentrbl. Bakteriol. 1997, 285(3), 368-378.

**Segal, E.D., S. Falkow and L.S. Tompkins.** (1996) Helicobacter pylori attachmentto gastric cells induces cytoskeletal rearrangements and tyrosine phosphorylation of host cell proteins. Proc Natl Acad Sci U S A **93:** 1259-1264.

**Sigmund, C. D., M. Ettayebi, and E. A. Morgan.** 1988. Antibiotic resistance mutations in ribosomal RNA genes of Escherichia coli. Methods Enzymol. **164:**673-690.

**Sipponen, P. and Hyvärinen, H.** (1993) Role of *Helicobacter pylori* in the pathogenesis of gastritis, peptic ulcer and gastric cancer. *Scand J Gastroenterol* **28:** 3-6.

**Solnick, J.V., J. O'Rourke, A. Lee, B.J. Paster, F.E. Dewhirst, and L.S. Tompkin.** 1993. An uncultured gastric spiral organism is a newly identified Helicobacter in humans. J. Infect. Dis. **168:**379-385.

**Sorberg, M., M. Nilsson, H. Hanberger and L.E. Nilsson.** (1996) Morphologic conversion of Helicobacter pylori from bacillary to coccoid form. Eur J Clin Microbiol Infect Dis **15:** 216-219.

**Stolte, M. and Eidt, S.** (1993) Healing gastric MALT lymphomas by eradicating *H pylori*. *Lancet* **342:** 568

**Stopler, T. and D. Branski.** 1986. Resistance of Mycoplasma pneumoniae to macrolides, lincomycin, and streptograminB. J. Antimicrob. Chemother. **18:**359-364.

**Taylor, D.N. and Blaser, M.J.** (1991) The epidemiology of *Helicobacter pylori* infection. *Epidemiol Rev* **13:** 42-59.

**Trebesius K., D. Harmsen, A. Rakin, J. Schmelz and J. Heesemann.** 1998. Development of rRNA targeted PCR and in situ hybridization with fluorescently labelled oligonucleotides for detection of *Yersinia* species. J. Clin. Microbiol. **36,** in press.

**Vester, B., and R. A. Garrett.** 1987. A plasmid-coded and site-directed mutation in Escherichia coli 23S rRNA that confers resistance to erythromycin: implications for the mechanism of action of erythromycin. Biochimie **69:**891-900.

**Versalovic, J., M. S. Osato, K. Spakovsky, M. P. Dore, R. Reddy, G. G. Stone, D. Shortridge, R. K. Flamm, S. K. Tanaka and D. Y. Graham.** 1997. Point mutations in the 23S rRNA gene of Helicobacter pylori associated with different levels of clarithromycin resistance. J. Antimicrob. Chemotherapy. **40:** 283-286.

**Wagner, M., R. Amann, H. Lemmer, and K. H. Schleifer.** 1993. Probing activated sludge with proteobacteria-specific oligonucleotides: inadequacy of culture-dependent methods for describing microbial community structure. Appl. Environ. Microbiol. 59:1520-1525.

**Wagner, M., G. Rath, R. Amann, H.-P. Koops, and K.-H. Schleifer.** 1995. In situ identification of ammonia-oxidizing bacteria. System. Appl. Microbiol. **18:** 251-264.

**Wahl, G.M., S.L. Berger and A.R. Kimmel.** (1987) Molecular hybridization of immobilized nucleic acids;theoretical concepts and practical considerations. Methods in Enzymology **152**:399-407.

**Wang, X., E. Sturegard, R. Rupar, H.O. Nilsson, P.A. Aleljung, B. Carlen, R. Willen and T. Wadström.** (1997) Infection of BALB/c A mice by spiral and coccoid forms of Helicobacter pylori. J Med Microbiol **46:** 657-663.

**Warren, J.R. and Marshall, B.** (1983) Unidentified curved bacilli on gastric epithelium in active chronic gastritis. *Lancet* i: 1273-1275.

**Weisblum, B.** 1995. Erythromycin resistance by ribosome modification. Antimicrob. Agents Chemother. **39**:577-585.

**Westblom, T.U., E. Madan, and B.R. Midkiff.** 1991. Egg yolk emulsion agar, a new medium for the cultivation of *Helicobacter pylori*. Journal of Clinical Microbiology **29:** 819-821.

**Woese, C. R.** 1987. Bacterial evolution. Microbiol. Rev. **51**:221-271.

**Zuckerkandl, E., and L. Pauling.** 1965. Molecules as documents of evolutionary history. J. Theoret. Biol. **8:**357-366

## Patentansprüche

1. Verfahren zum Nachweis von Makrolid-Antibiotikumresistenzen bei Mikroorganismen umfassend die Schritte:
a) Bereitstellen einer Mikroorganismen enthaltenden Probe,
b) Inkontaktbringen der Probe mit mindestens einer Hybridisierungssonde, die spezifisch für eine mit Makrolid-Antibiotikumresistenzen assoziierte Nukleinsäuresequenz in Mikroorganismen ist, unter Bedingungen, die eine spezifische Hybridisierung der Sonde erlauben und
c) Auswerten der Probe in situ durch Bestimmung des Auftretens oder des Ausbleibens einer Hybridisierung.

2. Verfahren nach Anspruch 1,
**dadurch gekennzeichnet,**
**daß** die Mikroorganismen aus bakteriellen Keimen und Protozoen ausgewählt werden.

3. Verfahren nach Anspruch 1 oder 2,
**dadurch gekennzeichnet,**
**daß** die mit Makrolid-Antibiotikumresistenzen assoziierte Nukleinsäuresequenz aus ribosomalen Nukleinsäuresequenzen ausgewählt wird.

4. Verfahren nach Anspruch 3,
**dadurch gekennzeichnet,**
**daß** die Nukleinsäuresequenz aus bakteriellen 23 S ribosomalen Nukleinsäuresequenzen ausgewählt wird.

5. Verfahren nach Anspruch 4,
**dadurch gekennzeichnet,**
**daß** die Nukleinsäuresequenz einen Bereich entsprechend einem oder mehreren der Nukleotide 2032, 2057, 2058, 2059, 2503 und 2611 auf der E.coli 23S rRNA umfaßt.

6. Verfahren nach einem der vorhergehenden Ansprüche,
**dadurch gekennzeichnet,**
**daß** man langsam wachsende oder/und in vitro schwierig oder nicht kultivierbare Pathogene testet.

7. Verfahren nach Anspruch 6,
**dadurch gekennzeichnet,**
**daß** die Mikroorgansimen gewählt werden aus der Gruppe bestehend aus Helicobacter spec, Mycobacterien, Porphyromonas gingivalis, Propionibacterium acnes, Borrelia burgdorferi, Mycoplasmen, Chlamydien, Tropheryma whippelii, Bartonellen Legionellen, Norkardien und Actinomyceten.

8. Verfahren nach einem der vorhergehenden Ansprüche,
**dadurch gekennzeichnet,**
**daß** man eine aus menschlichen oder tierischen Geweben oder Körperflüssigkeiten stammende Probe verwendet.

9. Verfahren nach einem der vorhergehenden Ansprüche,
**dadurch gekennzeichnet,**
**daß** die Probe ohne vorherige Kultivierung der Mikroorganismen untersucht wird.

10. Verfahren nach einem der vorhergehenden Ansprüche,
**dadurch gekennzeichnet,**
**daß** die Probe einer Anreicherungsprozedur für Mikroorganismen unterzogen wird.

11. Verfahren nach einem der vorhergehenden Ansprüche,
**dadurch gekennzeichnet,**
**daß** die Probe vor der Untersuchung mit einem Presumptive-Medium versetzt wird.

12. Verfahren nach Anspruch 11,
**dadurch gekennzeichnet,**
**daß** das Presumptive-Medium eine Indikatorsubstanz zur Typisierung von Mikroorganismen enthält.

13. Verfahren nach einem der vorhergehenden Ansprüche,
**dadurch gekennzeichnet,**
**daß** die Probe vor der Untersuchung fixiert und gegebenenfalls permeabilisiert wird.

14. Verfahren nach einem der vorhergehenden Ansprüche,
**dadurch gekennzeichnet,**
**daß** die Hybridisierungssonde aus Nukleinsäuren wie DNA oder Nukleinsäureanaloga wie PNA ausgewählt wird.

15. Verfahren nach einem der vorhergehenden Ansprüche,
**dadurch gekennzeichnet,**
**daß** die Hybridisierungssonde einen Hybridisierungsbereich mit einer Länge entsprechend 10 bis 30 Nukleotidbausteinen, bevorzugt 15 bis 20 Nukleotidbausteinen, insbesondere 17 bis 18 Nukleotidbausteinen aufweist.

16. Verfahren nach einem der vorhergehenden Ansprüche,
**dadurch gekennzeichnet,**
**daß** man eine Hybridisierungssonde verwendet, die spezifisch für Mutationen ausgewählt aus Deletionen, Transversionen, Transitionen und Modifikationen der entsprechenden Wildtypsequenz ist.

17. Verfahren nach einem der vorhergehenden Ansprüche,
**dadurch gekennzeichnet,**
**daß** man eine Kombination von mehreren Hybridisierungssonden verwendet, die spezifisch für unterschiedliche mit Antibiotikumresistenzen assoziierten Nukleinsäuresequenzen sind.

18. Verfahren nach einem der Ansprüche 16 oder 17,
**dadurch gekennzeichnet,**
**daß** man die Hybridisierungssonden ClaR1 (SEQ ID NO. 1), ClaR2 (SEQ ID NO. 2) oder/und ClaR3 (SEQ ID NO. 3) verwendet.

19. Verfahren nach einem der vorhergehenden Ansprüche,
**dadurch gekennzeichnet,**
**daß** man zusätzlich mindestens eine Hybridisierungssonde verwendet, die spezifisch für eine mit einem Wildtyp des Mikroorganismus assoziierte Nukleinsäuresequenz ist.

20. Verfahren nach Anspruch 19,
**dadurch gekennzeichnet,**
**daß** man die Hybridisierungssonde ClaWT (SEQ ID NO. 4) verwendet.

21. Verfahren nach einem der vorhergehenden Ansprüche,
**dadurch gekennzeichnet,**
**daß** man zusätzlich mindestens eine Hybridisierungssonde verwendet, die spezifisch für eine Spezies oder Gattung von Mikroorganismus ist.

22. Verfahren nach Anspruch 21,
**dadurch gekennzeichnet,**
**daß** man zum Nachweis von Helicobacter pylori Hybridisierungs-sonden verwendet, die gegen Sequenzen aus H.pylori 16S rRNA gerichtet sind, die homolog zu den E.coli Regionen 110-140, 740-780, 585-605 oder/und 210-245 sind.

23. Verfahren nach Anspruch 22,
**dadurch gekennzeichnet,**
**daß** man die Hybridisierungssonden Hpyl-165-753 (SEQ ID NO. 5), 120b (SEQ ID NO. 6), 585 (SEQ ID NO. 7) oder/und 219 (SEQ ID NO.8) verwendet.

24. Verfahren nach Anspruch 21,
**dadurch gekennzeichnet,**
**daß** man zum Nachweis von Helicobacter heilmannii Hybridisierungssonden verwendet, die gegen Sequenzen aus H.heilmanii 16S rRNA gerichtet sind, die homolog zu den E.coli Regionen 580-610 oder/und 640 - 670 sind.

25. Verfahren nach Anspruch 24,
**dadurch gekennzeichnet,**
**daß** man die Hybridisierungssonden Hh1 (SEQ ID NO. 9), Hh2 (SEQ ID NO. 10), Hh3 (SEQ ID NO. 11) oder/und Hh4 (SEQ ID NO. 12) verwendet.

26. Verfahren nach einem der vorhergehenden Ansprüche,
**dadurch gekennzeichnet,**
**daß** man Hybridisierungssonden verwendet, die eine Direktmarkierung tragen.

27. Verfahren nach einem der vorhergehenden Ansprüche,
**dadurch gekennzeichnet,**
**daß** man Hybridisierungssonden verwendet, die mit Farbstoff-, Fluoreszenz- oder/und Enzymgruppen markiert oder markierbar sind.

28. Verfahren nach einem der vorhergehenden Ansprüche,
**dadurch gekennzeichnet,**
**daß** man mehrere Hybridisierungssonden verwendet, die unterschiedlich markiert oder markierbar sind.

29. Verfahren nach einem der vorhergehenden Ansprüche,
**dadurch gekennzeichnet,**
**daß** die Auswertung der Probe durch mikroskopische Methoden erfolgt.

30. Verfahren nach einem der vorhergehenden Ansprüche,
**dadurch gekennzeichnet,**
**daß** die Auswertung eine quantitative Bestimmung von Makrolid-Antibiotikumresistenzen umfaßt.

31. Verwendung eines in situ Nukleinsäure-Hybridisierungsverfahrens zum Nachweis von Makrolid-Antibiotikumresistenzen in Mikroorganismen.

32. Verwendung nach Anspruch 31 zum Nachweis von Makrolid-Antibiotikumresistenzen in Bakterien oder Protozoen.

33. Verwendung nach Anspruch 32 zum Nachweis von Makrolid-Antibiotikumresistenzen ausgewählt aus der Gruppe bestehend aus Clarithromycin, Erythromycin, Azithromycin und Roxithromycin.

34. Reagenzienkit zum Nachweis von Makrolid-Antibiotikumresistenzen in Mikroorganismen durch in situ Hybridisierung umfassend
(a) Mittel zur Probenvorbereitung und
(b) mindestens eine Hybridisierungssonde, die spezifisch für eine mit Makrolid-Antibiotikumresistenzen assoziierte Nukleinsäuresequenz in Mikroorganismen ist, und gegebenenfalls mindestens ein Mittel zur Typisierung von Mikroorganismen.

35. Reagenzienkit nach Anspruch 34,
**dadurch gekennzeichnet,**
**daß** die Mittel zur Probenvorbereitung ein Presumptive-Medium und gegebenenfalls Anreicherungsmittel für Mikroorganismen umfassen.

36. Reagenzienkit nach Anspruch 35,
**dadurch gekennzeichnet,**
**daß** das Presumptive-Medium eine Nährlösung mit einer Stickstoffquelle und weiteren essentiellen Komponenten sowie gegebenenfalls reduzierenden Substanzen oder/und Sauerstoff-abweisenden Zusätzen enthält.

37. Reagenzienkit zum Nachweis von Makrolid-Antibiotikumresistenzen in Mikroorganismen umfassend
(a) ein Presumptive-Medium für Mikroorganismen und
(b) mindestens eine Hybridisierungssonde, die spezifisch für eine mit Makrolid-Antibiotikumresistenzen assoziierte Nukleinsäuresequenz in Mikroorganismen ist und gegebenenfalls Mittel zur Typisierung von Mikroorganismen.

38. Reagenzienkit nach Anspruch 37,
**dadurch gekennzeichnet,**
**daß** das Presumptive-Medium eine Nährlösung mit einer Stickstoffquelle und weiteren essentiellen Komponenten sowie gegebenenfalls reduzierenden Substanzen oder/und Sauerstoff-abweisenden Zusätzen enthält.

39. Reagenzienkit nach Anspruch 37 oder 38,
**dadurch gekennzeichnet,**
**daß** die Mittel zur Typisierung von Mikroorganismen Indikatorsubstanzen umfassen, die im Presumptive-Medium gelöst oder/und suspendiert sind.

40. Reagenzienkit nach Anspruch 39,
**dadurch gekennzeichnet,**
**daß** er einen Ureaseindikator zum Nachweis von Helicobacter spec., insbesondere von H.pylori oder/und H.heilmannii enthält.

41. Verwendung eines Reagenzienkits nach einem der Ansprüche 34 bis 40 in einem Verfahren nach einem der Ansprüche 1 bis 30.

42. Verwendung nach Anspruch 41,
**dadurch gekennzeichnet,**
**daß** die Mittel zur Typisierung von Mikroorganismen ein Oligonukleotid aus einem Bereich der V-Domäne der 16S rRNA zum Spezies-spezifischen Nachweis von Helicobacter, insbesondere von H. pylori oder/und H.heilmannii, umfassen.

43. Verwendung nach Anspruch 42,
**dadurch gekennzeichnet,**
**daß** das Oligonukleotid die in SEQ ID NO. 5, 6, 7, 8, 9, 10, 11 oder/und 12 dargestellte Sequenz oder zumindest einen 10 Nukleotide langen Teilbereich der Sequenz enthält.

44. Verwendung nach Anspruch 42 oder 43,
**dadurch gekennzeichnet,**
**daß** das Oligonukleotid eine Markierungsgruppe trägt.

45. Verwendung nach Anspruch 41,
**dadurch gekennzeichnet,**
**daß** die Hybridisierungssonde, die spezifisch für eine mit Makrolid-Antibiotikumrestistenzen assoziierte Nukleinsäuresequenz in Mikroorganismen ist, ein Oligonukleotid aus einer bakteriellen 23S rRNA umfaßt.

46. Verwendung nach Anspruch 45,
**dadurch gekennzeichnet,**
**daß** das Oligonukleotid die in SEQ ID NO. 1, SEQ ID NO. 2 oder SED ID NO. 3 dargestellte Sequenz enthält.

47. Verwendung nach Anspruch 45 oder 46 zusammen mit einem Wildtyp-spezifischen Oligonukleotid, insbesondere einem Oligonukleotid, das die in SEQ ID NO. 4 dargestellte Sequenz enthält.

48. Oligonukleotid,
**dadurch gekennzeichnet,**
**dass** es die in SEQ ID NO. 1, 2, 3 oder 4 dargestellte Sequenz oder zumindest einen 10 Nukleotide langen Teilbereich der Sequenz enhält.

49. Oligonukleotid nach Anspruch 48,
**dadurch gekennzeichnet,**
**daß** es eine Markierungsgruppe trägt.

## Claims

1. Method for detecting macrolide antibiotic resistances in microorganisms comprising the steps:
a) preparing a sample containing microorganisms,
b) contacting the sample with at least one hybridization probe which is specific for a nucleic acid sequence associated with macrolide antibiotic resistances under conditions which allow a specific hybridization of the probe and
c) analysing the sample in situ by determining the occurrence or absence of a hybridization.

2. Method as claimed in claim 1,
**characterized in that**
the microorganisms are selected from bacterial organisms and protozoa.

3. Method as claimed in claim 1 or 2,
**characterized in that**
the nucleic acid sequence associated with macrolide antibiotic resistances is selected from ribosomal nucleic acid sequences.

4. Method as claimed in claim 3,
**characterized in that**
the nucleic acid sequence is selected from bacterial 23 S ribosomal nucleic acid sequences.

5. Method as claimed in claim 4,
**characterized in that**
the nucleic acid sequence encompasses a region corresponding to one or more of the nucleotides 2032, 2957, 2058, 2059, 2503 and 2611 on the E. coli 23S rRNA.

6. Method as claimed in one of the previous claims,
**characterized in that**
slowly growing pathogens or/and pathogens which are difficult to culture or cannot be cultured in vitro are tested.

7. Method as claimed in claim 6,
**characterized in that**
the microorganisms are selected from the group comprising Helicobacter spec., mycobacteria, Porphyromonas gingivalis, Propionibacterium acnes, Borrelia burgdorferi, mycoplasmas, chlamydias, Tropheryma whippelii, bartonellas, legionellas, nocardias and actinomycetes.

8. Method as claimed in one of the previous claims,
**characterized in that**
a sample is used which is derived from human or animal tissues or body fluids.

9. Method as claimed in one of the previous claims,
**characterized in that**
the sample is examined without prior culture of the microorganisms.

10. Method as claimed in one of the previous claims,
**characterized in that**
the sample is subjected to a procedure for concentrating microorganisms.

11. Method as claimed in one of the previous claims,
**characterized in that**
a presumptive medium is added to the sample before the examination.

12. Method as claimed in claim 11,
**characterized in that**
the presumptive medium contains an indicator substance for typing microorganisms.

13. Method as claimed in one of the previous claims,
**characterized in that**
the sample is fixed before the examination and optionally permeabilized.

14. Method as claimed in one of the previous claims,
**characterized in that**
the hybridization probe is selected from nucleic acids such as DNA or nucleic acid analogues such as PNA.

15. Method as claimed in one of the previous claims,
**characterized in that**
the hybridization probe has a hybridization region having a length corresponding to 10 to 30 nucleotide building blocks, preferably 15 to 20 nucleotide building blocks in particular 17 to 18 nucleotide building blocks.

16. Method as claimed in one of the previous claims,
**characterized in that**
a hybridization probe is used which is specific for mutations selected from deletions, transversions, transitions and modifications or the corresponding wild type sequence.

17. Method as claimed in one of the previous claims,
**characterized in that**
a combination of several hybridization probes is used which are specific for different nucleic acid sequences associated with antibiotic resistances.

18. Method as claimed in one of the claims 16 or 17,
**characterized in that**
the hybridization probes ClaR1 (SEQ ID NO. 1), ClaR2 (SEQ ID NO. 2) or/and ClaR3 (SEQ ID NO. 3).

19. Method as claimed in one of the previous claims,
**characterized in that**
at least one hybridization probe is additionally used which is specific for a nucleic acid sequence which is associated with a wild type of the microorganism.

20. Method as claimed in claim 19,
**characterized in that**
the hybridization probe ClaWT (SEQ ID NO. 4) is used.

21. Method as claimed in one of the previous claims,
**characterized in that**
at least one hybridization probe is additionally used which is specific for a microorganism species or genus.

22. Method as claimed in claim 21,
**characterized in that**
hybridization probes are used to detect Helicobacter pylori which are directed against sequences from H. pylori 16S rRNA and are homologous to the E. coli regions 110-140, 740-780, 585-605 or/and 210-245.

23. Method as claimed in claim 22,
**characterized in that**
the hybridization probes Hpyl-165-753 (SEQ ID NO. 5), 120b (SEQ ID NO. 6), 585 (SEQ ID NO. 7) or/and 219 (SEQ ID NO. 8) are used.

24. Method as claimed in claim 21,
**characterized in that**
hybridization probes are used to detect Helicobacter heilmannii which are directed against sequences from H. heilmannii 16S rRNA which are homologous to the E. coli regions 580-610 or/and 640-670.

25. Method as claimed in claim 24,
**characterized in that**
the hybridization probes Hh1 (SEQ ID NO. 9), Hh2 (SEQ ID NO. 10), Hh3 (SEQ ID NO. 11) or/and hh4 (SEQ ID NO. 12) are used.

26. Method as claimed in one of the previous claims,
**characterized in that**
hybridization probes are used which carry a direct label.

27. Method as claimed in one of the previous claims,
**characterized in that**
hybridization probes are used which are labelled or can be labelled with dye groups, fluorescence groups or/and enzyme groups.

28. Method as claimed in one of the previous claims,
**characterized in that**
several hybridization probes are used which have different labels or can be labelled differently.

29. Method as claimed in one of the previous claims,
**characterized in that**
the sample is analysed by microscopic methods.

30. Method as claimed in one of the previous claims,
**characterized in that**
the analysis comprises a quantitative determination of macrolide antibiotic resistances.

31. Use of an in situ nucleic acid hybridization method to detect macrolide antibiotic resistances in microorganisms.

32. Use as claimed in claim 31 to detect macrolide antibiotic resistances in bacteria or protozoa.

33. Use as claimed in claim 32 to detect macrolide antibiotic resistances selected from the group comprising clarithromycin, erythromycin, azithromycin and roxithromycin.

34. Reagent kit for detecting macrolide antibiotic resistances in microorganisms by in situ hybridization comprising
(a) means for sample preparation and
(b) at least one hybridization probe which is specific for a nucleic acid sequence associated with macrolide antibiotic resistances in microorganisms and optionally at least one means for typing microorganisms.

35. Reagent kit as claimed in claim 34,
**characterized in that**
the means for sample preparation comprise a presumptive medium and optionally means for concentrating microorganisms.

36. Reagent kit as claimed in claim 35,
**characterized in that**
the presumptive medium contains a nutrient solution containing a nitrogen source and other essential components and optionally reducing substances or/and oxygen-repelling additives.

37. Reagent kit for detecting macrolide antibiotic resistances in microorganisms comprising
(a) a presumptive medium for microorganisms and
(b) at least one hybridization probe which is specific for a nucleic acid sequence associated with macrolide antibiotic resistances in microorganisms and optionally at least one means for typing microorganisms.

38. Reagent kit as claimed in claim 37,
**characterized in that**
the presumptive medium contains a nutrient solution containing a nitrogen source and other essential components and optionally reducing substances or/and oxygen-repelling additives.

39. Reagent kit as claimed in claim 37 or 38,
**characterized in that**
the means for typing microorganisms comprise indicator substances which are dissolved or/and suspended in the presumptive medium.

40. Reagent kit as claimed in claim 39,
**characterized in that**
it contains a urease indicator for detecting Helicobacter spec. and in particular H. pylori or/and H. heilmannii.

41. Use of a reagent kit as claimed in one of the claims 34 to 40 in a method as claimed in one of the claims 1 to 30.

42. Use as claimed in claim 41,
**characterized in that**
the means for typing microorganisms comprise an oligonucleotide from a region of the V domain of the 16S rRNA for the species-specific detection of Helicobacter, in particular of H. pylori or/and H. heilmannii.

43. Use as claimed in claim 42,
**characterized in that**
the oligonucleotide contains the sequence shown in SEQ ID NO. 5, 6, 7, 8, 9, 10, 11 or/and 12 or a section of the sequence which is at least 10 nucleotides long.

44. Use as claimed in claim 42 or 43,
**characterized in that**
the oligonucleotide carries a labelling group.

45. Use as claimed in claim 41,
**characterized in that**
the hybridization probe which is specific for a nucleic acid sequence in microorganisms which is associated with macrolide antibiotic resistances comprises an oligonucleotide from a bacterial 23S rRNA.

46. Use as claimed in claim 45,
**characterized in that**
the oligonucleotide contains the sequence shown in SEQ ID NO. 1, SEQ ID NO. 2 or SEQ ID NO. 3.

47. Use as claimed in claim 45 or 46 together with a wild type-specific oligonucleotide, in particular an oligonucleotide which contains the sequence shown in SEQ ID NO.4.

48. Oligonucleotide,
**characterized in that**
it contains the sequence shown in SEQ ID NO. 1, 2 or 3 or a section of the sequence which is at least 10 nucleotides long.

49. Oligonucleotide as claimed in claim 48,
**characterized in that**
it carries a labelling group.

## Revendications

1. Procédé pour l'identification de résistances à des antibiotiques du type macrolides dans des microorganismes, comprenant les étapes qui consistent en :
a) la mise à disposition d'un échantillon contenant des microorganismes,
b) la mise en contact de l'échantillon avec au moins une sonde d'hybridation qui est spécifique pour une séquence d'acides nucléiques associée à des résistances à des antibiotiques macrolides dans les microorganismes, sous des conditions qui permettent une hybridation spécifique de la sonde et
c) l'évaluation de la sonde in situ par détermination de l'apparition ou de l'absence d'une hybridation.

2. Procédé selon la revendication 1,
**caractérisé**
**en ce que** les microorganismes sont choisis parmi des germes et des protozoaires bactériens.

3. Procédé selon la revendication 1 ou 2,
**caractérisé**
**en ce que** la séquence d'acides nucléiques associée à des résistances aux antibiotiques macrolides est choisie parmi des séquences d'acides nucléiques ribozomiques.

4. Procédé selon la revendication 3,
**caractérisé**
**en ce que** la séquence d'acides nucléiques est choisie parmi des séquences d'acides nucléiques ribozomiques bactériennes 23 S.

5. Procédé selon la revendication 4,
**caractérisé**
**en ce que** la séquence d'acides nucléiques comprend une zone correspondant à un ou plusieurs des nucléotides 2032, 2057, 2058, 2059, 2503 et 2611 de l'ARNr de E.coli 23S.

6. Procédé selon l'une des revendications précédentes,
**caractérisé**
**en ce que** l'on teste des pathogènes à croissance lente ou/et à culture difficile ou non cultivables in vitro.

7. Procédé selon la revendication 6,
**caractérisé**
**en ce que** les microorganismes sont choisis dans le groupe constitué par helicobacter spec, les mycobactéries, porphyromonas gingivalis, propionibacterium acnés, borrelia burgdorferi, les mycoplasmes, les chlamydiae, tropheryma whippelii, les bartonelles les legionelles, norkardies et l'actinomycétène.

8. Procédé selon l'une des revendications précédentes,
**caractérisé**
**en ce que** l'on utilise un échantillon provenant de tissus humains ou animaux ou de fluides corporels.

9. Procédé selon l'une des revendications précédentes,
**caractérisé**
**en ce que** l'échantillon est examiné sans mise en culture préalable des microorganismes.

10. Procédé selon l'une des revendications précédentes,
**caractérisé**
**en ce que** l'échantillon est soumis à un procédé d'enrichissement pour microorganismes.

11. Procédé selon l'une des revendications précédentes,
**caractérisé**
**en ce que**, avant l'examen, un milieu présomptif est ajouté à l'échantillon.

12. Procédé selon la revendication 11,
**caractérisé**
**en ce que** le milieu présomptif contient une substance indicative pour déterminer le type de microorganismes.

13. Procédé selon l'une quelconque des revendications précédentes,
**caractérisé**
**en ce que** l'échantillon est fixé avant l'examen et éventuellement perméabilisé.

14. Procédé selon l'une des revendications précédentes,
**caractérisé**
**en ce que** la sonde d'hybridation est choisie parmi les acides nucléiques tels que l'ADN ou les analogues de l'acide nucléique tels que l'APN.

15. Procédé selon l'une des revendications précédentes,
**caractérisé**
**en ce que** la sonde d'hybridation présente un domaine d'hybridation d'une longueur correspondant à 10 jusqu'à 30 modules de nucléotides, de préférence 15 à 20 modules de nucléotides, en particulier 17 à 18 modules de nucléotides.

16. Procédé selon l'une des revendications précédentes,
**caractérisé**
**en ce que** l'on utilise une sonde d'hybridation qui est choisie spécifiquement pour les mutations parmi les délétions, transversions, transitions et modifications de la séquence de type sauvage correspondante.

17. Procédé selon l'une des revendications précédentes,
**caractérisé**
**en ce que** l'on utilise une combinaison de plusieurs sondes d'hybridation qui sont spécifiques pour différentes séquences d'acides nucléiques associées à des résistances à des antibiotiques.

18. Procédé selon l'une des revendications 16 ou 17,
**caractérisé**
**en ce que** l'on utilise les sondes d'hybridation ClaR1 (SEQ ID NO. 1), ClaR2 (SEQ ID NO. 2) ou/et ClaR3 (SEQ ID NO. 3).

19. Procédé selon l'une des revendications précédentes,
**caractérisé**
**en ce que** l'on utilise, de plus, au moins une sonde d'hybridation qui est spécifique pour une séquence d'acides nucléiques associée à un type sauvage du microorganisme.

20. Procédé selon la revendication 19,
**caractérisé**
**en ce que** l'on utilise la sonde d'hybridation ClaWT (SEQ ID NO. 4).

21. Procédé selon l'une des revendications précédentes,
**caractérisé**
**en ce que** l'on utilise, de plus, au moins une sonde d'hybridation qui est spécifiqueme pour une espèce ou variété de microorganisme.

22. Procédé selon la revendication 21,
**caractérisé**
**en ce que** pour l'identification d'helicobacter pylori on utilise des sondes d'hybridation qui sont dirigées contre les séquences de H. pylori 16S ARNr, qui sont homologues aux régions E.coli 110-140, 740-780, 585-605 ou/et 210-245.

23. Procédé selon la revendication 22,
**caractérisé**
**en ce que** l'on utilise les sondes d'hybridation. Hpyl-165-753 (SEQ ID NO. 5), 120b (SEQ ID NO. 6), 585 (SEQ ID NO. 7) ou/et 219 (SEQ ID NO. 8).

24. Procédé selon la revendication 21,
**caractérisé**
**en ce que**, l'identification d'helicobacter heilmannii, on utilise des sondes d'hybridation qui sont dirigées contre des séquences de h.heilmannii 16S ARNr, qui sont homologues aux régions e.coli 580-610 ou/et 640-670.

25. Procédé selon la revendication 24,
**caractérisé**
**en ce que** l'on utilise les sondes d'hybridation Hh1 (SEQ ID NO. 9), Hh2 (SEQ ID NO. 10), Hh3 (SEQ ID NO. 11) ou/et Hh4 (SEQ ID NO. 12).

26. Procédé selon l'une des revendications précédentes,
**caractérisé**
**en ce que** l'on utilise des sondes d'hybridation qui portent un marquage direct.

27. Procédé selon l'une des revendications précédentes,
**caractérisé**
**en ce que** l'on utilise des sondes d'hybridation qui sont marquées ou peuvent être marquées par des groupes de colorants, de fluorescence ou/et d'enzymes.

28. Procédé selon l'une des revendications précédentes,
**caractérisé**
**en ce que** l'on utilise plusieurs sondes d'hybridation qui sont marquées ou peuvent être marquées de façon différente.

29. Procédé selon l'une des revendications précédentes,
**caractérisé**
**en ce que** l'évaluation de l'échantillon s'effectue par des procédés microscopiques.

30. Procédé selon l'une des revendications précédentes,
**caractérisé**
**en ce que** l'évaluation comprend une détermination quantitative des résistances aux antibiotiques macrolides dans des microorganismes.

31. Utilisation d'un procédé in situ d'hybridation d'acides nucléiques pour l'identification de résistances aux antibiotiques macrolides dans des microorganismes.

32. Utilisation selon la revendication 31 pour l'identification de résistances aux antibiotiques macrolides dans les bactéries ou protozoaires.

33. Utilisation selon la revendication 32 pour l'identification de résistances aux antibiotiques macrolides choisies à partir du groupe constitué par la clarithromycine, l'érythromycine, l'azithromycine et la roxithromycine.

34. Kit d'agents réactifs pour l'identification de résistances à des antibiotiques macrolides dans des microorganismes par hybridation in situ comprenant
(a) des moyens pour la préparation de l'échantillon et
(b) au moins une sonde d'hybridation qui est spécifique pour une séquence d'acides nucléiques associée à des résistances à des antibiotiques macrolides dans des microorganismes et, éventuellement, au moins un moyen pour la détermination du type de microorganismes.

35. Kit d'agents réactifs selon la revendication 34,
**caractérisé**
**en ce que** les moyens pour la préparation de l'échantillon comprennent un milieu présomptif et, éventuellement, un milieu d'enrichissement pour microorganismes.

36. Kit d'agents réactifs selon la revendication 35,
**caractérisé**
**en ce que** le milieu présomptif contient une solution nutritive avec une source d'azote et d'autres composants essentiels, ainsi qu'éventuellement des substances réductrices ou/et des additifs rejetant l'oxygène.

37. Kit d'agents réactifs pour la mise en évidence de résistances aux antibiotiques macrolides dans des microorganismes comprenant
(a) un milieu présomptif pour les microorganismes et
(b) au moins une sonde d'hybridation qui est spécifique pour une séquence d'acides nucléiques associée à des résistances aux antibiotiques macrolides dans des microorganismes et, éventuellement, des moyens pour déterminer le type de microorganismes.

38. Kit d'agents réactifs selon la revendication 37,
**caractérisé**
**en ce que** le milieu présomptif contient une solution nutritive comprenant une source d'azote et d'autres composants essentiels, ainsi qu'éventuellement des substances réductrices ou/et des additifs repoussant l'oxygène.

39. Kit d'agents réactifs selon la revendication 37 ou 38,
**caractérisé**
**en ce que** les moyens pour la détermination du type de microorganismes comprennent des substances du type indicateur qui sont dissoutes ou/et mises en suspension dans le milieu présomptif.

40. Kit d'agents réactionnels selon la revendication 39,
**caractérisé**
**en ce qu'**il contient un indicateur d'uréase pour l'identification d'helicobacter spec., en particulier d'H.pylori ou/et H.heilmannii.

41. Utilisation d'un kit de réactifs selon l'une des revendications 3.4 à 40 dans un procédé selon l'une des revendications 1 à 30.

42. Utilisation selon la revendication 41,
**caractérisée**
**en ce que** les moyens pour la détermination du type de microorganisme comprennent un oligonucléotide provenant d'une plage du domaine v de 16S ARNr pour la mise en évidence ou identification spécifique de l'espèce d'helicobacter, en particulier d'H. pylori ou/et H.heilmannii.

43. Utilisation selon la revendication 42,
**caractérisée**
**en ce que** l'oligonucléotide contient la séquence représentée dans SEQ ID NO. 5, 6, 7, 8, 9, 10, 11 ou/et 12 ou au moins une zone partielle de la séquence d'une longueur de 10 nucléotides.

44. Utilisation selon la revendication 42 ou 43,
**caractérisée**
**en ce que** l'oligonucléotide porte un groupe de marquage.

45. Utilisation selon la revendication 41,
**caractérisée**
**en ce que** la sonde d'hybridation, qui est spécifique pour une séquence d'acides nucléiques associée aux résistances à des antibiotiques macrolides dans des microorganismes, comprend un oligonucléotide provenant d'un ARNr 23S bactérien.

46. Utilisation selon la revendication 45,
**caractérisée**
**en ce que** l'oligonucléotide contient la séquence représentée dans SEQ ID NO. 1, SEQ ID NO. 2 ou SEQ ID NO. 3.

47. Utilisation selon la revendication 45 ou 46, conjointement avec un oligonucléotide spécifique à l'égard du type sauvage, en particulier un oligonucléotide qui contient la séquence représentée dans SEQ ID NO. 4.

48. Oligonucléotide,
**caractérisé**
**en ce qu'**il contient la séquence représentée dans SEQ ID NO. 1, 2 ou 3 ou au moins une zone partielle de la séquence d'une longueur de 10 nucléotides.

49. Oligonucléotide selon la revendication 48,
**caractérisé**
**en ce qu'**il porte un groupe de marquage.


### SEQUENZPROTOKOLL

<110> Creatogen Biosciences GmbH
<120> Nachweis von Antibiotikumresistenzen in Mikroorganismen
<130> DE 198 23 098.2
<140> 9678956
<141> 1998-05-22
<160> 12
<170> PatentIn Ver. 2.1
<210> 1
<211> 17
<212> DNA
<213> Helicobacter pylori A2058G (ClaR)
<400> 1
   cggggtcttc ccgtctt 17

<210> 2
<211> 17
<212> DNA
<213> Helicobacter pylori A2059G
<400> 2
   cggggtctct ccgtctt 17

<210> 3
<211> 17
<212> DNA
<213> Helicobacter pylori A2058C (ClaR)
<400> 3
   cggggtcttg ccgtctt 17

<210> 4
<211> 17
<212> DNA
<213> Helicobacter pylori Wildtyp
<400> 4
   cggggtcttt ccgtctt 17

<210> 5
<211> 18
<212> DNA
<213> Helicobacter pylori
<400> 5
   gctttcgcgc aatcagcg 18

<210> 6
<211> 18
<212> DNA
<213> Helicobacter pylori
<400> 6
   aggcacatga tctatgcg 18

<210> 7
<211> 21
<212> DNA
<213> Helicobacter pylori
<400> 7
   cacacctgac tgactatccc g 21

<210> 8
<211> 22
<212> DNA
<213> Helicobacter pylori
<400> 8
   ggacataggc tgatctctta gc 22

<210> 9
<211> 20
<212> DNA
<213> Helicobacter heilmannii
<400> 9
   cccacactcc agaagratag 20

<210> 10
<211> 21
<212> DNA
<213> Helicobacter heilmannii
<400> 10
   cccacactct agggttkgca g 21

<210> 11
<211> 20
<212> DNA
<213> Helicobacter heilmannii
<400> 11
   cccacactct agaaagatag 20

<210> 12
<211> 21
<212> DNA
<213> Helicobacter heilmannii
<400> 12
   cacatctgac ttgccacccc g 21